# EUROPEAN PATENT SPECIFICATION

(11) **EP 2 893 351 B1**
(45) Date of publication and mention of the grant of the patent: **06.12.2023**
(21) Application number: 13776424.7
(22) Date of filing: 09.09.2013
(51) Int. Cl.: G01N 33/574

(54) **METHOD FOR IDENTIFYING SUBGROUPS OF CIRCULATING TUMOR CELLS (CTCS) IN THE CTC POPULATION OF A BIOLOGICAL SAMPLE**
VERFAHREN ZUR IDENTIFIZIERUNG VON UNTERGRUPPEN ZIRKULIERENDER TUMORZELLEN IN DER CTC-POPULATION EINER BIOLOGISCHEN PROBE
PROCÉDÉ D'IDENTIFICATION DE SOUS-GROUPES DE CELLULES TUMORALES CIRCULANTES (CTC) DANS LA POPULATION DE CTC D'UN ÉCHANTILLON BIOLOGIQUE

(30) Priority: 07.09.2012 EP 12183492
(43) Date of publication of application: 15.07.2015
(73) Proprietor: Hoffmann, Andreas-Claudius, 45239 Essen (DE); Schlaak, Jörg Friedrich, 45131 Essen (DE)
(72) Inventor: Hoffmann, Andreas-Claudius, 45239 Essen (DE); Schlaak, Jörg Friedrich, 45131 Essen (DE)
(74) Representative: Simandi, Claus
(86) International application number: PCT/EP2013/068558
(87) International publication number: WO 2014/037552

(56) References cited:
- WO-A1-2011/093927
- WO-A1-2013/082163
- Thomas Niemetz: "Phenotypic and molecular characterization of colorectal cancer-derived circulating and disseminated tumor cells", Dissertation, 11 June 2012 (2012-06-11), pages 1-119, XP002693369, Heidelberg, DE Retrieved from the Internet: URL:http://archiv.ub.uni-heidelberg.de/vol ltextserver/13422/1/niemietzthesisfinal.pd f [retrieved on 2013-03-07]
- NEL IVONNE ET AL: "A novel method for the detection of different subgroups of circulating tumor cells in patients with hepatocellular carcinoma", HEPATOLOGY, vol. 56, no. Suppl. 1, 1 October 2012 (2012-10-01), page 457A, XP002693370, & 63RD ANNUAL MEETING OF THE AMERICAN-ASSOCIATION-FOR-THE-STUDY-OF-LIVE R-DISEASES (AASLD); BOSTON, MA, USA; NOVEMBER 09 -13, 2012
- YUN-FAN SUN ET AL: "Circulating stem cell-like epithelial cell adhesion molecule-positive tumor cells indicate poor prognosis of hepatocellular carcinoma after curative resection", HEPATOLOGY, 4 March 2013 (2013-03-04), pages n/a-n/a, XP055055494, ISSN: 0270-9139, DOI: 10.1002/hep.26151

## Description

The invention relates to a method for identifying and characterizing subpopulations (subgroups) of circulating tumor cells (CTCs) in a population of CTCs in a biological sample, preferably in a blood sample of the patient. Hepatocellular carcinoma (HCC) currently is the fifth most common malignancy worldwide with an annual incidence up to 500 per 100000 individuals depending on the geographic region investigated. Whereas 80% of new cases occur in developing countries, the incidence increases in industrialized nations including Western Europe, Japan and the United States (El-Serag HB, N.Engl.J.Med. 1999; 340:745-750). To manage patients with HCC, tumor markers are very important tools for diagnosis, evaluation of disease progression, outcome prediction and evaluation of treatment efficacy. Several tumor markers have been reported for HCC, which include α-fetoprotein (AFP) (Di Bisceglie AM J Hepatol 2005), Lens culinaris agglutinin-reactive fraction of AFP (AFP-L3) (OKA H, J Gastroenteroll Hepatol 2001), and des- γ-carboxy prothrombin (DCP) (Liebman HA N Engl J Med 1984). However, none of these tumor markers show 100% sensitivity or specificity, which calls for new and better biomarkers.

In order to identify novel biomarkers of HCC, many clinical studies utilizing omics-based methods have been reported over the past decade. In particular, the proteomics-based approach has turned out to be a promising one, offering several quantification techniques to reveal differences in protein expression that are caused by a particular disease. In the most studies reported in literature, the well-established 2D-DIGE (two-dimensional difference in gel electrophoresis) technique has been applied for protein quantification followed by identification via mass spectrometry.

Curative therapeutic options are limited to early stages and include mostly resection, sometimes orthotopic liver transplantation (LTx) if patients present with cirrhosis (3, 4). High recurrence rates after resection and even liver transplantation, most likely due to minimal residual disease (5, 6) and the fact that most patients are diagnosed in an advanced stage make palliative approaches necessary. The treatment of choice in a palliative setting is local administration of chemotherapy or radiotherapy. A selective internal radiation therapy (SIRT) employing 90mYttrium-loaded microsphere has become a major part of this local approach (7, 8). Up to now there are only very few possibilities to monitor treatment success. CT-scans often need several weeks to show an effect of local therapies, leaving the patient at risk of progressing unnoticed and hence limiting further options due to the effect of progressing liver disease. Furthermore, there are still no reliable markers of a relapse after primary tumor resection or LTx. Currently available serum based markers like Alpha-fetoprotein (AFP), Des-Gamma-Carboxyprothrombin (DCP) or the Lectin 3-Fraction of AFP (AFP-L3) are incapable of predicting the clinical outcome with high accuracy and reproducibility (9). Tissue derived molecular markers seem to be a promising approach to individualize patients' therapy but lack the possibility of monitoring the patient during or after treatment, since this would require ongoing biopsies and hence increased risks for the patient. Therefore, the development of minimal invasive diagnostic methods is imperative to characterize the tumor at the molecular level, to find sensitive markers for monitoring of postoperative recurrence as well as to evaluate therapy response and consequently to give adequate treatment for HCC patients.

Circulating tumor cells (CTC) in the peripheral blood of patients with HCC may be the solution to this diagnostic dilemma as a so-called "liquid biopsy". They already have been described in breast and lung cancer (10-12). Up to now only a few reports have been published on the isolation of CTC in HCC patients using indirect methods like qRT-PCR or direct visualization of circulating epithelial cells (13-17). So far CTC detection methods consist of enrichment and subsequent identification, with immunomagnetic bead selection based on antibodies for tumor cell surface antigens being currently the most common method.

Epithelial cell adhesion molecule (EpCAM) is widely expressed on the surface of epithelial cells and epithelial derived tumor cells (18). Therefore EpCam positive CTC were quantified and correlated with patients' outcome to examine their use in the clinical setting (12, 19). Mahreswaran and colleagues showed that CTC were also applicable for molecular approaches during therapy like the detection of EGFR mutations in patients with Non-small cell lung cancer (20). Identification of circulating tumor cells is often done with anti-cytokeratin antibodies, since blood of healthy individuals contains few or no cytokeratin-positive cells (21). In recent years the epithelial-to-mesenchymal-transition (EMT) has gained more attention since tumor cells may downregulate both EpCAM and CK to gain invasiveness and metastatic potential and potentially resistance to ongoing systemic treatment (22, 23). Therefore, focusing on epithelial cell surface markers is not sufficient for detecting a heterogeneous population of CTC including those with a mesenchymal phenotype.

WO 2011/093927 A1 discloses methods for dectecting CTCs in a biological sample. The method includes the detection of least one epithelial mesenchymal transition (EMT) biomarker. In this method EpCAM positive cells are isolated by the Cell Search System (Veridex, Raritan, NJ) from blood samples and further investigated without prior enrichment of CTCs from the sample.

Since CTCs are very rare and hard to find in a strong background of leucocytes, often less than one in 10⁶ peripheral blood mononucleated cells (PBMC), sophisticated approaches are needed for CTC detection.

Thomas Niemetz ("Phenotypic and molecular characterization of colorectal cancer-derived circulating and disseminating tumor cells", Dissertation (11.06.2012), XP002693369, Heidelberg) discloses several methods for the enrichment of CTCs. The direct enrichment of CTCs from blood spiked with HT29 CRC cells with anti-EpCAM-coated magnetic beads was more efficient after densitiy gradient centrifugation compared to a direct use in whole blood, but in general this method was considered to be inefficient due to strong leukocyte carry-over. In another approach CD45 was used to deplete leukocytes in blood samples and to enrich a CTC fraction. Also with this method leukocyte carry-over was too strong. Than FACS sorting was considered to isolate CTCs out of leukocyte-depeleted and EpCAM/CD45 stained blood samples. Subsequent analysis by quantitative PCR revealed that these objects were probably unspecific fluorescent signals or no intact cells. Since cell sorting by flow cytometry was not successful and bears the risk of losing the rare CTCs, it was decided to apply a micromanipulator for CTC isolation. After densitiy centrifugation and anti-CD45 bead based pre-enrichment, CTCs were identified in stained blood samples by fluorescence microscopy by using EpCAM-directed antibodies. Single cells were analyzed by fluorescence microscopy.

It is obvious, that the enrichment of CTCs from a biological sample is difficult since they are very rare and because of their cellular heterogeneity and plasticity.

The problem to be solved is to provide an easy and reliable marker and method for diagnosis and prognosis for individuals having tumors, in particular for solid tumors like for example HCC and non-small cell lung cancer.

The present invention provides a method for studying biological samples for tumors, namely metastatic renal cell carcinoma (mRCC). and non-small cell lung cancer. Hereto, the present invention provides a method to identify and charcterize circulating tumor cells (CTCs) in a biological sample and to align these CTCs to subgroups. Thereby the CTC subgroup composition of a biological sample can be determined and changes in the CTC subgroup composition of a sample e.g. from a particular patient can be detected. The CTC subgroups identified by the method of the invention can be used as biomarkers for cancer and tumors. The novel method of the inventions characterizes subtypes/subgroups of circulating tumor cells (CTCs) that are characteristic for example for the patient, the tumor, the stage and the aggressiveness of the tumor. The mixture of subgroups (subtypes) of CTCs in a population of CTCs in a biological sample is identified by the method and can be used as a reliable biomarker.

The CTCs comprised in a population of CTCs of a biological sample can be assigned to one or more different subgroups (subpopulations) of CTCs, e.g. CTCs that are in the epithelial stage or CTCs that are in the mesenchymal stage or CTC that display features of the epithelial stage and the menchymal stage. With the method of the invention the different CTC subgroups can be identified, this could be only one or two or several different subgroups of CTCs. The method according to the invention allows monitoring changes in the composition and/or the ratio of CTC subgroups in a population of CTCs.

In addition, the method is suitable for the routine analysis and monitoring of tumor patients, since it is easy and reproducible to perform due to the fact, that CTCs are enriched from the biological sample prior to the analysis of CTC subgroup composition and / or CTC subgroup concentration. The method is suitable for qualitative as well as quantitative analysis of CTC subgroups in a biological sample, in particular, if the conditions of enrichment are performed in comparison to a standard or control sample.

The present invention relates to a method for analyzing the composition of circulating tumor cell (CTC) population in a biological sample from an individual for use in the diagnosis or prognosis of a tumor comprising the steps
a) isolation or enrichment of pheriperal blood mononuclear cells (PMBCs) and CTCs from the biological sample,
b) depleting hematopoietic cells from the isolated or enriched PMBCs and CTCs,
c) testing the obtained cell suspension from step b) for at least two markers independently from each other selected from epithel markers, wherein the epithel markers are selected from the group comprising epithelial cell adhesion molecule (EpCAM), epithelial growth factor receptor (EGFR), cytokeratin (CK), pan-cytokeratin (Pan-CK), E-Cadherin,
   and/or
   mesenchymal markers, wherein the mesenchymal markers are selected from the group comprising N-Cadherin and Vimentin,
      and/or
   tumor markers,wherein the tumor markers are selected from the group comprising AFP, KRT18, KRT19, TGFb, HIF1a, IGFBP1, IGFBP5, IGF, HGFAC, APC, VEGFA, PDGFA, FGFA, HIF1A, KDR1 (VEGFR), FGF2, HBsAg, ASGPR1, aSMA, Hep-Par-1,
      and/or
   markers for resistance namely C-Met,
      and/or
   stem cell markers namely CD133,
      and/or
   hematopoietic markers, wherein the hematopoietic marker is selected from the group comprising CD45 or CD15,
      and
d) thereby identifying one or more subgroup(s) of CTCs in the CTC population of that biological sample, wherein the composition of subgroup(s) of CTCs is characteristic for a specific tumor, wherein the tumor is selected from the group of non-small cell lung cancer (NSCLC), metastatic renal cell carcinoma (mRCC).

In particular, the method comprising the step of isolating or enrichment of PBMCs and CTCs from the biological sample prior to depletion of hematopoietic cells.

Further, additional markers can be applied in order to identify CTC subgroups. This embodiment comprises the step of testing the obtained cell suspension for at least one additional marker selected from epithel markers, mesenchymal markers, epithel-to-mesenchymal transition (EMT) markers, surface markers, tissue markers, tumor markers, markers for resistance, stem cell markers, hematopoietic markers.

A biological sample can comprise a population of CTCs. A population of CTCs can comprise one or more subgroups of CTCs. The composition of subgroup(s) of circulating tumor cells (CTCs) is characteristic for a specific tumor, for example a specific solid tumor. CTCs subpopulations and the respective subgroups of CTCs are therefore surrogate markers for cancer, tumors and /or metastasis. The subgroups of CTCs are in addition charcteristic for a particular individual, for example a patient. The individual subgroup CTC composition in the biological sample is specific for a particular patient. It can be used to predict and monitor treatment and disease progression or regression respectively. The CTC subgroups and the respective amounts (ratio) in the population of CTCs or the sample that are identified by the method of the invention can therefore be used as specific biomarker for the respective tumor, the therapeutic treatment and the individual patient, these biomarkes are for example suitable for individualized medicine.

In another preferred embodiment the method comprises the step of isolating or enrichment of peripheral blood mononuclear cells (PBMC) and CTCs from the biological sample prior to depletion of hematopoietic cells, for example by densitiy gradient centrifugation. In a preferred embodiment of the invention PMBCs and CTCs are isolated or enriched by densitiy gradient centrifugation.

However, it is not necessary that the separation is complete. In preferred embodiment of the invention at least 50 % of the PBMCs of the original sample, e.g. the peripheral blood serum of the patient, are separated from the blood sample in order to obtain a cell suspension depleted from hematopoetic cells.

In another embodiment of the method hematopoietic cells are depleted using anti-CD45 and/or anti-CD15 antibodies, for example immunomagnetic beads coated with anti-CD45 and/or anti-CD15 antibodies. In the method according to the invention hematopoietic cells are depleted from the population of CTCs or the sample, e.g. the blood sample with anti-CD45 coated immunomagnetic beads. In a preferred embodiment a mixture of beads, preferably immunomagnetic beads, linked with CD45 and CD15 antibodies are used.

According to the invention for example 55 to 85 %, preferably 65 to 75 %, most preferred about 70 % of the hematopoietic cells are depleted from the population of CTCs or the sample, e.g. the blood sample.

In another preferred embodiment the method according to the invention comprises the step of enrichment of epithelial cells after the CD45-depletion step. The enrichment of epithelial cells in the cell suspension can be performed for example with anti-EpCAM antibodies. The anti-EpCAM antibodies are for example bound to immunomagnetic beads. Cells bearing EpCAM are bound to anti-EpCAM antibodies and can than easily be separated from the cell suspension be removing the immunomagnetic beads.

In another preferred embodiment of the invention the biological sample is taken from one individual. The individual can be a human, preferably a patient. In another embodiment the individual can be any mammal. In a preferred embodiment of the invention the method for studying a biological sample is characterized in that the sample is a human sample.

In another embodiment of the method the epithel markers are selected from the group comprising epithelial cell surface markers, epithelial cell adhesion molecule (EpCAM), epithelial growth factor recepctor (EGFR), cytokeratin (CK), pan-cytokeratin (Pan-CK), E-Cadherin, and/or
the mesenchymal markers are selected from the group comprising N-Cadherin and Vimentin and/or
the EMT markers are selected from the group comprising SNAIL, Twist, N-Cadherin, C-Met and/or
the tissue markers or tumor markers are selected from AFP, KRT18, KRT19, TGFb, HIF1a, IGFBP1, IGFBP5, IGF, HGFAC, APC, VEGFA, PDGFA, FGFA, HIF1A, KDR1 (VEGFR), FGF2, HBsAg, ASGPR1, aSMA, Hep-Par-1 and/or
the marker for resistance is selected from C-Met and/or
the stem cell marker is selected from CD133 and/or
the hematopoietic marker is selected from CD45 or CD15.

Preferably EpCAM is used as epithel marker.

Cells can in addition be stained with DAPI in order to identify undamaged cells.

In another embodiment of the method the presence of the marker(s) in the CTC subgroup is/are detected by immunofluorescence, PCR or FISH.

In a preferred embodiment of the invention the markers (epithelial markers, mesenchymal markers, tissue markers, tumor markers, stem cell markers, resistance markers indicating for example resistance a particular treatment or pharmaceutical) are detected by immunofluorescence. Different subpopulations (subgroups) of CTCs can than for example be detected by counter staining. Individual profiles of patients, of a particular tumor, of a particular stage of tumor, of tumor progression or regression, of monitoring of treatment are for example created by counter staining thereby detecting individual characteristic subgroups of one or more CTCs. The method according to the invention thereby allows the determination of the composition of the CTC population at a certain point of time. This can be used for example for prognosis and therapeutic strategy. In addition, the method is suitable to monitore the changes in the composition of the CTC population during time, for example during teatment or after treatment.

The invention further relates to the use of the method for diagnosis of a tumor, like for example for detection of a tumor in an individual, for monitoring tumor development like for example for prognosis, for pediction of the risk for metastasis, for prediction of the progression free survival, for evaluation of treatment like for example for predicition of responsiveness of the individual having a tumor to a particular treatment, for monitoring of treatment like for example for monitoring progression of the tumor in an individual undergoing therapeutic treatment or for monitoring progression of the tumor in an individual without treatment, for disease control like for example for determination of the stage of epithelial-to-mesenchymal-transition (EMT), for characterization of the tumor type, for the identification and/or validation of pharmaceutical compositions or pharmaceutical compounds that are useful for treatment of a particular individual.

Therefore, the invention realtes to the use of the method wherein the tumor is selected from non-small cell lung cancer (NSCLC), metastatic renal cell carcinoma (mRCC).

In another embodiment the invention relates to the use of the method for determining the ratio of one subgroup of CTCs to another subgroup of CTCs in the CTC population of the biological sample of an individual, for example to determine the ratio of mesenchymal CTCs to epithelial CTCs.

It further realtes to the use of the method for determining the change of composition of CTC subgroups in the CTC population of an individual over time comprising the step of collecting a biological sample from an individual at least at two different points of time, analyzing the composition of the respective CTC populations in these biological samples and comparing the respective CTC subgroup compositions.

In a preferred embodiment of the invention the sample is a blood sample, for example blood serum, blood plasma, whole blood, a biopsy sample. The sample than comprises many PBMCs which are separated from the rest of the cell suspension.

In another preferred embodiment of the invention the method comprises the step of testing the cell suspension/the CTCs for one or more markers for epithelial-to-mesenchymal-transition (EMT). Markers for EMT are for example N-Cadherin and/ or c-Met.

The invention further relates to a method according to the invention for identifying a subgroup of circulating tumor cell (CTC) associated with non-small cell lung cancer comprising the steps
a) enrichment of mononuclearic cells including CTCs from a blood sample,
b) depletion of hematopoietic cells from the blood sample,
c) use of one or more of markers to identify non-small cell lung cancer specific CTC subgroup(s), for example one or more marker(s) selected from the markers Pan-CK, CD133 and N-Cadherin.

Disclosed is a method for identifying a subgroup of circulating tumor cell (CTC) associated with mRCC comprising the steps
a) enrichment of mononuclearic cells including CTCs from a blood sample,
b) depletion of hematopoietic cells from the blood sample,
c) use of one or more of markers to identify metastatic renal cell carcinoma specific CTC subgroup(s), for example one or more marker(s) selected from the markers CK, EpCAM, N-cadherin, CD133, CD45, LDH, Kreatin.

A quantification of the CTCs in subgroups is for example possible using Immunofluoresenz-labelling or PCR. It therefore allows to dermine and monitore the amount and / or ratio of different subgroups of CTCs in a CTC population or a sample.

Also disclosed is the use of the method according to the invention, for example for characterization of tumors, namely non-small cell lung cancer and/or mRCC.

The disclosure relates to the continous monitoring of patients and/or sample composition with respect to CTC subgroups.

The disclosure further relates to the use of the method for determining the ratio of mesenchymal CTCs to epithelial CTCs in the sample or the cell suspension respectively. The invention in this connection also relates to the use of the method for predicting the progression free survival. The aggressiveness of the tumor, the responsiveness to treatment and the prognosis can be determined by the ratio of mesenchymal CTCs to epithelial CTCs. A ratio of mesenchymal CTCs to epithelial CTCs (e.g. determined by cells expressing N-Cadherin+/CK+) lower than 0,5, preferably lower than 0,2, most preferred lower than 0,1 resembles for expampe an association to shortened progression free survival. Therefore, the invention further relates to the use of the method for predicting aggressiveness of tumor, progression free survival (PFS), disease control, selection of treatment, surveillance of treatment, response to treatment, outcome of treatment, metastasis and recurrence.

The disclosure further relates to the use of the method for determining the stage of epithelial-to-mesenchymal-transition (EMT) of the respective tumor.

The disclosure further relates to the use of the method for diagnosis. The following aspects of a diagnostic / test kit or device are not according to the invention and are present for illustration purposes only. The method further relates to the use of a diagnostic device or a test kit for the analysis of tumor specific CTCs in a sample. The method further relates to a diagnostic device or a test kit for the enrichment and detection of tumor specific CTCs. Such diagnostic test or test kit for performing a method according to the invention may comprising means for depleting hematopoietic cells from a blood sample and means for detection of one or more markers selected form epithelial markers, mesenchymal markers and / or tumor markers. Such a diagnostic device or test kit may comprise means for the detection of the ratio of different subtypes of CTCs in a blood sample. A diagnostic device or test kit may for example comprise means for the detection of EpCAM. A diagnostic device or test kit may comprise means for the immunological detection of the respective markers of CTC subgroups, preferably for their immunfluorescence detection. The method further relates to a diagnostic device or test kit for analysing the amount of CTC subgroups comprising at least one agent for the specific detection of one or more marker(s) associated with EMT, epithelial stage, mesenchymal stage and the respective tumor and wherein the diagnostic device or test kit further comprises detection reagents and further aids.

The present disclosure further relates to a method for studying a biological sample for the respective tumor, wherein the sample is studied for one or more marker(s) for epithel cells, mesenchymal cells and/or tumor and wherein the marker(s) is / are differentially expressed in relation to the healthy state indicating the presence of the tumor.

In one embodiment of the method the differential expression of the particular marker in the respecitive CTC (sub-)population is determined by comparing the amount of this marker in a biological sample of a person without the disease with the amount of this protein in a person with the disease.

The present disclosure further relates to the use of the CTC subgroups identified by the method according to the invention for screening pharmaceutical compounds for the respective tumor and / or the respective patient.

The present disclosure further relates to a screening assay comprising one or more CTC subgroups identified by the method according to the invention and using theses CTC subgroups for the identification and validation of pharmaceutical compounds for a respective tumor and / or a respective patient and wherein the screening assay comprises detection reagents and further aids.

In the context of this invention, the term non-small cell lung cancer comprises any form of non-small cell lung cancer. The term is for example defined in Pschyrembel, Klinisches Wörterbuch [Clinical Dictionary], 263th edition, 2012, Berlin.

The disclosure, but not the claimed invention also comprises the use of isoforms of the respective markers. An "Isoform" of the respective protein is any of several different forms of the same protein. Different forms of a protein may be produced from related genes or may arise from the same gene by alternative splicing. A large number of isoforms are caused by single-nucleotide-polymorphisms or SNPs, small genetic differences between alleles of the same gene. These occur at specific individual nucleotide positions within a gene. Isoforms comprise also proteins with the same or similar amino acid sequence but different post-translational modification, like glycosylation. A glycoform is an isoform of a protein that differs only with respect to the number or type of attached glycan. Glycoproteins often consist of a number of different glycoforms, with alterations in the attached saccharide or oligosaccharide.

The disclosure, but not the claimed invention, also comprises homologes of the respective markers. A "homologe" of the respective protein is defined in terms of shared ancestry. Two segments of DNA can have shared ancestry because of either a specitation event (orthologs) or a duplication event (paralogs). The term "percent homology" and "sequence similarity" are used interchangeably. High sequence similarity might occur because of convergent evolution or because of chance. Such sequences are similar and are also included in the term according to the invention. Sequence regions that are homologous are also called conserved. Enclosed is also partial homology where a fraction of the sequences compared (are presumed to) share descent, while the rest does not. Many algorithms exist to cluster protein sequences into sequence families, which are sets of mutually homologous sequences, see for example databses HOVERGEN, HOMOLENS, HOGENOM. According to the invention homologes should display at least 80 % or 90 % or 95 % identity in amino acid sequence with the marker, preferably 96 % or 97 %, most preferably 98 % or 99 %.

In a preferred embodiment of the method according to the invention peripheral venous blood were drawn from patients with solid tumors, stored at room temperature and processed within 24 h after collection. Blood was diluted with PBS and layered into a tube, e.g. a Leucosep tube containing Ficoll-Paque below a porous barrier. After bouyant density gradient centrifugation (e.g. 1600 x g, 20 °C, 20 min) the interphase consisting of PBMNC and CTC can be removed and washed. For subtype analyses CTC were negatively enriched by hematopoietic cell depletion. For example, PBMNC were treated with a mixture of anti-CD45 and anti-CD15 or anti-CD45 or anti-CD15 alone coated immunomagnetic beads in a magnetic particle processor. The remaining cell suspension included bead-free pre-enriched tumor cells and is characterized by multi-immunofluorescence staining against at least two different markers and optionally nuclear counterstain with DAPI.

In several studies CTCs were enumerated and profiles of each tumor patient were examined. The total amount of N-cadherin-positive, CK-positive and CD133-positive cells and a ratio of mesenchymal to epithelial cells was conceived after negative enrichment using CD45/CD15-depletion. The enumerated potential CTC was normalized against the total PBMNC number and expressed the number of CTC per 1000 PBMNC. Individual cell type profiles were analyzed and correlated to therapeutic outcome.

In the following the embodiment HCC is not according to the invention and is present for illustration purposes only.

With this method a remarkable variation of cells with epithelial, mesenchymal, stem-cell and mixed characteristics such as N-cadherin+/CD45-, CK+/CD45-, N-cadherin+/CK+/CD45- and CD133+/CK+/CD45- was identified. In addition, it was found that cells stained positive for established CTC markers and CD45 such as CK+/EpCAM+/CD45+ as well as CK+/EpCAM-/CD45+. The distribution of CTC-subgroups varied significantly between different patient groups and was associated with therapeutic outcome. In NSCLC patients Kaplan-Meier-test showed that an increased number of N-cadherin+ cells prior admission of platinum-based therapy was significantly associated to shortened progression free survival (PFS; 8 vs 5 months; p=0.03; [HR]=2.8). In HCC-patients a N-cadherin+/CK+ ratio lower than 0.1 resembled an association to shortened time to progression (TTP; 1 vs 15 months; p=0.03; [HR]=0.19; 95% CI: 0.01-2.75. Spearman rank correlation showed that the number of CK+ cells prior to SIRT was sigInificantly associated to progression (p=0.01). Interestingly, the number of CK+ cells after SIRT was significantly correlated to the number of CD133+ cells. Furthermore, Mann-Whitney test revealed that CK+ cells (p=0.01) and CD133+ cells (p=0.12) before and after SIRT, respectively, differed significantly between patients with disease control and progression. In patients with mRCC that received anti-angiogenesis therapy Mann-Whitney test revealed that the number of N-cadherin+ cells was significantly lower in responders vs non-responders (p=0.04). Established clinical markers of response (LDH, Kreatinin and others) were included along with the mesenchymal to epithelial cell type ratio N-Cadherin/CK and total numbers of N-Cadherin+ and CD133+/CK- cells in a backward linear multivariate regression model. The overall model fit had a significance level of p=0.004 (<0.05). Ratio N-cadherin/CK (p=0.015), LDH (0.008) and Kreatinin (p=0.002) were the only factors in this patient cohort showing a significant independent correlation to response. Therefore, different CTC populations are identifiable in peripheral blood of patients with solid tumors and change during treatment courses and that these individual cell type profiles and their intraindividual changes may have distinct clinical implications. Rather the composition of cell types then the total amount of CTC is a potential clinical tool to predict and monitor clinical outcome. Furthermore, the data indicate cellular differences between entities regarding the process of EMT which has been reported to be associated with cancer aggressiveness.

With this invention a method was established to examine whether circulating tumor cells (CTC) and subtypes are identifiable in the peripheral blood of patients with cancer, in particular hepatocellular carcinoma.

Two strategies are applied to enrich and detect CTC in cancer, e.g. HCC. By one strategy CTC were negatively enriched by depleting hematopoietic cells using anti-CD45 immunomagnetic beads leading to a bead-free cell suspension. This cell suspension was then used for CTC type detection by immunofluorescence staining against various epithelial (e.g. Pan-CK, EpCAM) and mesenchymal markers (e.g. Vimentin, N-Cadherin) and one or more tissue specific marker(s), for example the liver specific ASGPR1.

Concomitant an equal part of this solution was further enriched after the depletion step using anti-EpCAM immunomagnetic beads resulting in a bead-bound EpCAM-positive CTC suspension. Identification of CTC in the EpCAM-positive bead-bound cell suspension was performed using immunocytochemical staining against Pan-CK or Hep Par 1, respectively with alkaline phosphatase. The CTC-enrichment step was confirmed by measuring mRNA expression levels of the epithelial genes EpCAM and Krt18 in depleted and enriched fractions.

To assess whether it is possible to identify and evaluate prognostic factors the genetic profile of CTCs in blood from patients during SIRTl was analyzed. The prognostic values of several candidate markers involved in carcinogenesis and liver disease, namely IGFBP1, HGFAC, Hifia, TGFb, APC, AFP, Hifia and Krt19 as well as their interrelationships was analyzed. The association of these gene expressions in EpCAM-positive CTC with response to SIRT using quantitative real-time RT-PCR in 25 patients with either disease control (partial response & stable disease) or progression was analyzed.

Therefore, two strategies were employed to enrich and detect CTC in HCC. Firstly, CTC was negatively enriched by depleting hematopoietic cells using anti-CD45 immunomagnetic beads leading to a bead-free CTC suspension which was used for CTC detection by immunofluorescence staining. Different populations of CTC were detected, such as epithelial cells that stained positive for pan-CK and DAPI but negative for EpCAM and CD45 as well as cells staining positive for both CK and EpCAM while negative for CD45. Also CTCs with mesenchymal properties such as vimentin and N-Cadherin as well as cells with both epithelial and mesenchymal features were detected. It has been described by Kalluri et al that CTC might undergo EMT and subsequently lose their epithelial features such as cell surface marker EpCAM and CKs (23). The detection of CTC showing mesenchymal phenotype was also reported by Gradilone et al. who assessed the prognostic value of CTC expressing mesenchymal markers in metastatic breast cancer patients (25, 26). They characterized CTC for CK and markers of EMT and found the gain of mesenchymal markers in CTC to be correlated to prognosis of patients in a follow-up of 24 months. Their data showed that the presence of mesenchymal markers on CTC more accurately predicted worse prognosis than the expression of CKs alone.

The data in the examples according to the invention demonstrate that the presence of mesenchymal cells correlates to survival. However, the results indicated that an increase in mesenchymal cells associates with better treatment outcome - hence that epithelial cells seem to be the driver of aggressiveness in this study population. Supporting these findings in the tested patients with HCC it was also revealed that the total amount of CK+ cells was higher in patients with a worse Child status. These contradicting results may be explainable by the different entity (breast vs. hepatocellular cancer) or different treatment approaches. Nearly all patients in this study had epithelial and mesenchymal cells in the peripheral blood, but in different proportions. Only some of these patients (4/11; 36%) presented cells with both features on the same cell. This indicates different stages of EMT. Cells were detected which stained positive for pan-CK and CD45 as well as for EpCAM, pan-CK and CD45. Cells with epithelial and hematological features were already described by Yu et al. (24). Since the identity of those cells is not yet clear they are generally excluded from the cell count in the clinically used methods, such as the CellSearch System (Veridex).

Nevertheless, this approach holds potential bias, since it has not yet been shown that these cells with additional CD45+ staining really are hematopoietic. Especially since the transfer of membrane proteins during contact known as trogocytosis can create novel cells with unique phenotype and altered function (27). In addition to this immunocytological variety of CTC distinct differences in size were observed. Commonly, CTC are described to have a large cellular size and a high nuclear to cytoplasmic ratio (14). In this study a wide range of signal intensities and a variety of cell sizes was found. There are a few reports though which confirm a significant variation of size within the same sample and different nuclear to cytoplasmic ratios (21, 28). Isolation by size of epithelial tumor cells (ISET) was applied by Vona et al. in patients with primary liver cancer (PLC) in order to evaluate its clinical impact. Fifty-two percent of total 44 PLC patients had detectable CTC, and their prevalence was significantly correlated with the presence of multifocal tumors, portal tumor thrombosis, and Child-Pugh class B/C (15). Hofman et al. compared ISET and CellSearch system with blood samples from NSCLC patients. Double immunostaining of the filters against CK and vimentin after ISET revealed circulating cells with non-hematopoietic features, but with cytological malignant criteria, which expressed only vimentin in a subpopulation of patients with no CTC detected by the CellSearch system (29).

The CellSearch system was applied by Schulze et al. to detect EpCAM-positive CTC in blood of HCC patients resulting in a detection rate of 45% (14 out of 31 HCC patients) and a significant correlation between the detection of CTC and pathological AFP values.(17) Xu et al. reported the development and validation of an EpCAM-independent magnetic cell separation system mediated by the interaction of the asialoglycoprotein receptor (ASGPR1) exclusively expressed on hepatocytes with its ligand. CTCs were then identified by Hep Par 1 staining. An average of 24 ± 19 CTCs per 5 ml blood was detected in 81% of HCC patient.

In the present invention negative CTC isolation was combined with immunofluorescence detection. Although hematologic cells were depleted using Anti-CD45 beads many cells remained in the suspension (log order 2 according to manufacturer's protocol; CD45 levels differ in leucocytes). The depletion rate was about 70% (data not shown). This method does not entirely deplete hematologic cells, but it offers an opportunity to detect the cells in a sample and to distinguish between different cell populations via counter staining and create an individual profile of each patient which might help anticipate patients' outcome. Since it is based on basic principles such as cellspin and immunolabeling it is an inexpensive procedure allowing quick clinical implementation. It is a cell size-independent method that includes, but is not solely dependent on EpCAM. The method of the present invention recognizes the fact that the total amount of hematopoietic as well as tumor cells varies significantly between patients and during treatment courses. Though the main goal of this study was to identify different CTC types, a ratio of these subtypes that seems to correlate well with therapeutic response was also established.

In order to extend the range of possible tumor cell enrichment it is necessary to have a system with greater flexibility to enrich and detect additional types of CTCs. The EpCAM-based enrichment method after density gradient centrifugation was comprehensively assessed by Guo et al who spiked healthy blood with a serial dilution of HepG2 cells and reported that as little as 10 cells in 5 ml blood could be detected using RT-PCR with primers specific for AFP. In clinical samples the positive detection rate ranged from 53 to 93% depending on the Child Pugh class (A, B and C) with a total positive detection rate of 73% (16). In the present study epithelial tumor cells seemed to be associated with worse outcome and more aggressive disease course. Therefore, epithelial cells were further enriched after the CD45-depletion step using anti-EpCAM immunomagnetic beads resulting in a bead-bound EpCAM-positive CTC suspension which still contained hematologic cells due to incomplete depletion. EpCAM- and KRT18 mRNA expression levels were compared in all cell fractions and was found to be significantly increased in the EpCAM-positive cell suspension compared to the CD45-positive hematopoietic cell fraction. These data revealed a specific enrichment of EpCAM-positive CTC employing anti-EpCAM immunomagnetic beads combined with a CD45 depletion step. Interestingly, KRT18 expression was significantly increased in the remaining bead-free cell suspension after CD45-depletion and after withdrawal of EpCAM-positive cells compared to the hematologic as well as to the EpCAM-positive cell fractions. The mRNA expression analysis data confirmed the occurrence of EpCAM-negative/KRT18-positive CTC that remain unbound in the residual cell fraction highlighting the results of the immunocytological staining. Consequently, the EpCAM enriched cell suspension was tested for potential molecular markers of therapeutic outcome choosing a set of genes involved in carcinogenesis and liver diseases.

In this invention a matched set of patients undergoing SIRT with either progression or partial response was used. The implementation of Spearman's test and the multivariate analysis illustrated a significant association of IGFBP1 (p=0.006) and AFP (p=0.04) mRNA expression with progression after SIRT (p=0.01). In this patient cohort however, AFP was excluded from the model when tested together with IGFBP1 mRNA expression (model fit p=0.01). After converting the continuous data to low and high expression groups with either progression or partial response (stable disease), low expression of IGBFP1 (p=0.01) and serological Bilirubin (p=0.02) were the only factors in this patient cohort showing a significant independent correlation to response. High AFP mRNA expression (p=0.0566) as well as serological AFP (p=0.0792) were by trend significant and Bilirubin (p=0.052) was significantly correlated to response. Interestingly, decreased IGFBP1 expression had a higher sensitivity (80%) and specificity (80%) than high AFP mRNA expression which has been reported as a well-recognized biomarker correlating with the metastasis and recurrence of HCC, and discussed to be the most useful molecular marker to prefigure the prognosis so far (16, 30). To validate the findings a second matched set of 11 HCC patients and repeated IGFPB1 mRNA expression analysis as described above was used. Spearman's test and the multivariate regression confirmed a significant correlation of IGFBP1 with progression after SIRT. The findings are in line with a study by Gong et al which revealed that IGFBP-1, 3 and 4 mRNA expression levels were significantly down regulated in HCC when compared to adjacent cirrhotic tissues and healthy livers which supports the concept that IGFBPs play an important inhibitory role in the development and/or growth of hepatocellular carcinoma (31).

In the present study IGFBP1 was also significantly associated with progression free survival in the Kaplan-Meier log rank test. ROC curve analysis indicated that changes in IGFBP1 mRNA expression ratios between early time points may help to evaluate therapy response or failure. Especially in liver cancer patients that do not benefit from treatment (e.g. SIRT) lose valuable time until re-evaluation of response using available imaging modalities.

With this invention different CTC populations are identifiable in peripheral blood of patients with HCC and it was shown that this individual cell type profile may have distinct clinical implications. Furthermore the data suggests that epithelial characteristics are associated with more aggressive tumors. The significant association of IGFBP1 mRNA expression to response in this study supports the use of CTC as biomarkers.

The following examples and figures are used to explain the invention without restricting the invention to the examples.

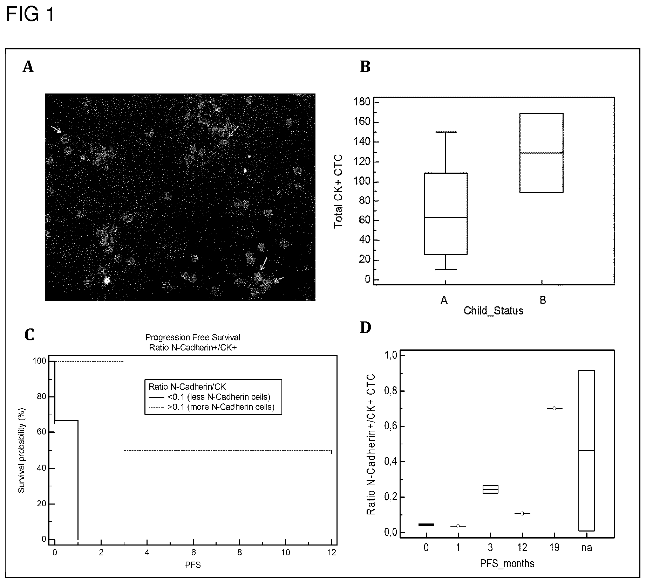

Fig. 1: Quantification of CTC subtypes and correlation with clinical characteristics. (A) Example of individual morphological profile showing 1 epithelial (CK-positive) and 3 mesenchymal (N-Cadherin-positive) CTC. (B) Amount of CK+ cells is associated to Child status. (C) Progression free survival vs. survival probability. (D) Ratio N-Cadherin+/CK+ cells is correlated to PFS (p=0.027).

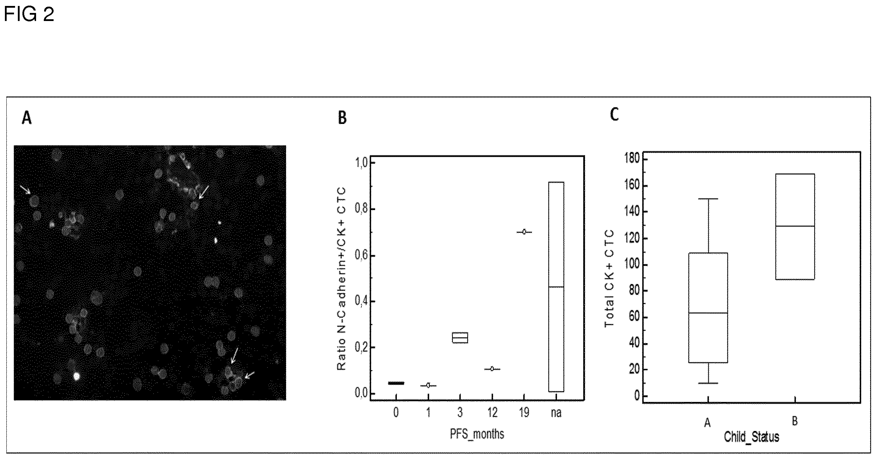

Fig. 2: CTC-enrichment. Detection of anti-EpCAM bead-bound CTC using immunochytochemical staining against (A) Pan-CK and (B) Hep-Par 1 with alkaline phosphatase. Gene expression levels of (C) EpCAM and (D) KRT18 in anti-EpCAM-bead-bound cells (EpCAM+), depleted hematologic cells (CD45+) and remaining bead-free cell suspension (Remain).

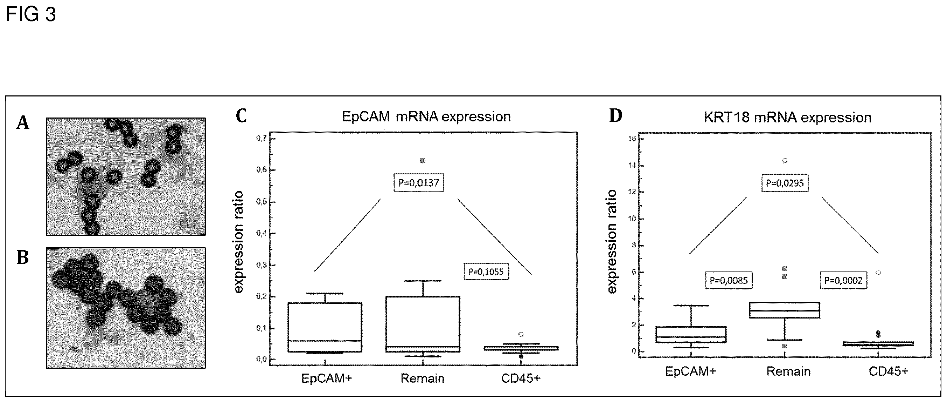

Fig. 3: Quantification of CTC subtypes and correlation with clinical characteristics. (A) Example of individual morphological profile showing 1 epithelial (CK-positive) and 3 mesenchymal (N-Cadherin-positive) CTC. (B) Ratio N-Cadherin+/CK+ cells is correlated to PFS (p=0.027). (C) Amount of CK+ cells is associated to Child status.

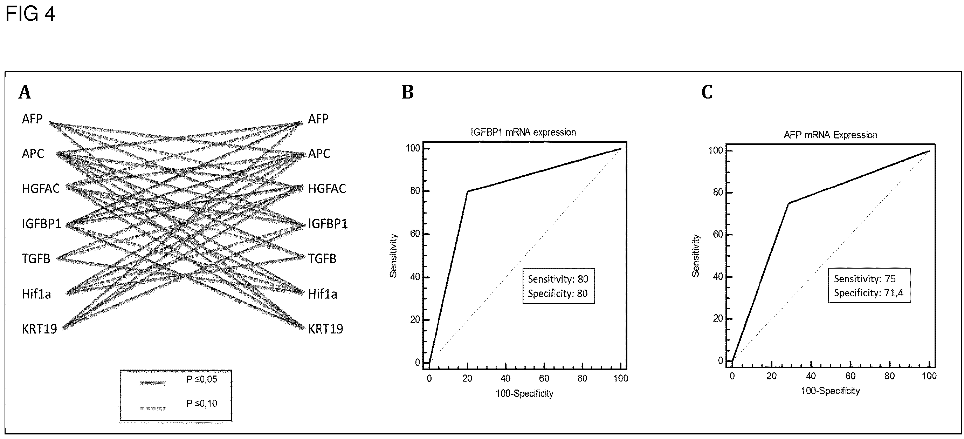

Fig 4: (A) Significant correlations between gene expressions measured in HCC patients. (B) ROC-curve analysis of IGFPB1 and (C) AFP.

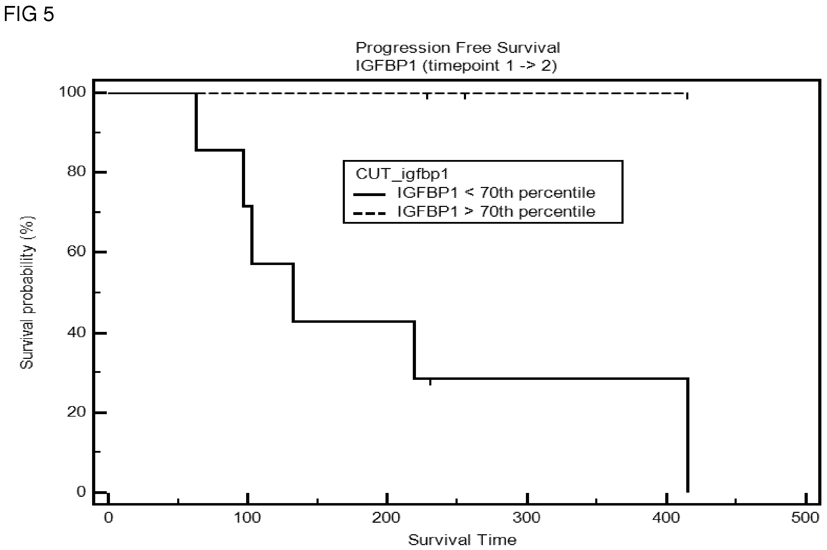

Fig. 5: Progression free survival vs. Survival probability.

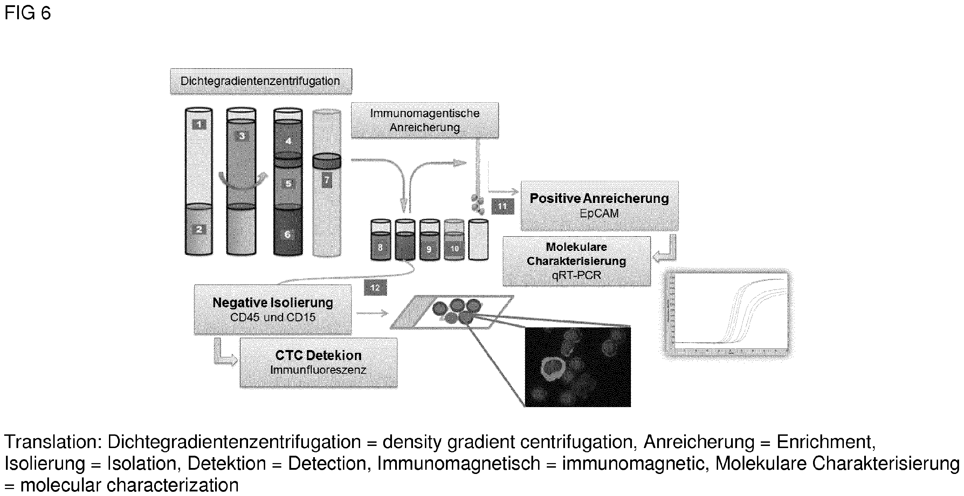

Fig. 6: Method according to the invention, schematic view.

### Examples

### Example 1: Materials and Methods

### Example 1a: Blood sample collection

40 ml citrated blood from HCC patients were collected, stored at room temperature and processed within 24 h after collection. Duplicates of 20 ml were used for sample preparation for subsequent CTC detection and gene expression analysis.

### Example 1b: Isolation of peripheral blood mononuclear cells (PBMC) using density gradient centrifugation

20 ml of citrated peripheral blood were diluted with 10 ml PBS and poured into a Leucosep tube (Greiner Bio-One) prepared with 16 ml Ficoll-Paque (GE-Healthcare) below the porous barrier. After density gradient centrifugation at 1600xg at 20°C for 20 min without break the PBMNC containing interphase above the barrier was transferred into a new tube, washed with 50 ml PBS containing 0.5% BSA and centrifuged at 300xg at 20 °C for 10 min. Subsequently, supernatant was removed completely.

### Example 1c: Sequential tumor cell enrichment using immunomagnetic beads

Negative isolation: Washed PBMC were resuspended in 1 ml of PBS containing 0.1% BSA and incubated with 25 µl anti-CD45 coated immunomagnetic Dynabeads (Invitrogen) for 30 min. Hematopoietic cells except erythrocytes, platelets and their precursor cells were bound to beads and separated by a magnetic particle processor (King Fisher mL, Thermo Fisher). The remaining cell suspension included bead-free pre-enriched tumor cells and was either subjected to immunocytochemical staining (see below) or subsequent positive epithelial tumor cell isolation.

Positive isolation: Cell suspension was incubated for 30 min with 50 µl FCR-blocking reagent (Miltenyi) and 10 µl immunomagnetic Epithelial Enrich-Dynabeads (Invitrogen) coated with a mouse IgG1 monoclonal EpCAM antibody. Tumor cells that are expressing EpCAM on their cell surfaces (EpCAM+ CTC) were bound to beads, separated by the magnet, washed and transferred into a new tube containing 100 µl PBS.

Positive and negative isolated CTC enriched suspensions were spun onto two glass slides, respectively, using a cytocentrifuge (Cell Spin II, Tharmac). After air drying cells were fixed in 96% Ethanol for 10 min and stored at 4-8°C until stained. For gene expression analyses a second sample was prepared in the same manner, but the CTC enriched suspension was centrifuged for 3 min at 350xg. After removing the supernatant cells were lysed with RLT lysis buffer (Qiagen) and samples were stored at -80°C until RNA extraction.

### Example 1d: Identification of CTC subtypes after negative isolation using immunocytochemical labeling

Immunofluorescence staining of epithelial and hematopoietic cells was carried out in the CD45-depleted pre-enriched tumor cell suspension. Briefly the staining method includes
a) fixation of the cells in 4,5% formaldehyde for 15 min, wash in PBS ,
b) permeabilization with 1x Perm/Wash Buffer (BD Biosciences) for 10 min, wash in PBS,
c) blocking of unspecific antibody reaction by incubation with blocking solution containing 5% BSA for 30 min,
d) binding of primary antibodies either ASGPR1 rabbit monoclonal antibody (ab42488, abcam) or pan-cytokeratin guinea pig polyclonal antibody (ABIN126062, antibodies-online) and EpCAM (E144) rabbit monoclonal antibody (ab32392, abcam) or vimentin (EPR3776) rabbit monoclonal antibody (2707-1, Epitomics) or N-Cadherin (EPR1792Y) rabbit monoclonal antibody (2019-1, Epitomics) for CTC and anti-CD45 (MEM-28) mouse monoclonal antibody (ab8216, abcam) for hematologic cells overnight at 4°C, wash in 0,1% Tween,
e) binding of secondary antibodies (FITC-conjugated AffiniPure goat anti-rabbit and Cy3-conjugated AffiniPure goat anti-mouse or AlexaFlour647-conjugated AffiniPure F(ab')2 Fragment goat anti-guinea pig; Jackson ImmunoResearch) for 30 min at 37°C, wash in 0,1% Tween.

Subsequently, cells were stained with 4,6-Diamidino-2-phenylindole dihydrochloride (DAPI) (Sigma-Aldrich) for 10 min, mounted with anti-fading medium (Invitrogen) and stored in the dark until evaluation. For each test a positive control slide with a mixture of human PBMNC and the hepatocellular carcinoma cell line HepG2 was treated under the same conditions. Microscopic evaluation was carried out using the digital Keyence BZ9000 (model BIOREVO) all-in-one fluorescence microscope with integrated camera and BZ-Analyzer Software. Stained slides were manually examined and objects that stained positive for DAPI and pan-cytokeratin or EpCAM or ASGPR1 or vimentin or N-Cadherin and negative for CD45 were captured and considered as tumor cells.

### Example 1e: Quantification of CTC subtypes

After negative CTC isolation each sample was split in half and processed onto glass slides for N-Cadherin/CK/CD45 and vimentin/CK/CD45 staining, respectively. Subsequently CTCs were detected within the same areas, each consisting of 10 visual fields using a 20x magnification on both slides.

Example 1f: Immunocytochemical staining for pan-cytokeratin-positive and Hep-Par-1-positive cells after EpCAM-positive tumor cell enrichment.

Detection of epithelial cells with the aid of immunofluorescence staining is technically impossible in the positively enriched CTC suspension due to light reflection by immunomagnetic beads. Therefore, identification of epithelial cells in the bead-bound enriched tumor cells suspension was performed using the UltraVision Quanto detection system AP (Thermo Scientific) according to the manufacturer's protocol. Briefly the staining method includes
a) permeabilisation with Perm/Wash buffer (BD Bioscience),
b) blocking of nonspecific background staining,
c) binding of primary mouse monoclonal pan-cytokeratin antibody-1 (Thermo Scientific, MS-343-R7) or Hep-Par-1 (Thermo Scientific, MS-1810-R7)
d) incubation with primary antibody amplifier Quanto (TL-125_QPB),
e) incubation with alkaline phosphatase-labeled polymer Quanto (TL-125-QAL) directed against mouse and rabbit antibodies,
f) visualization of polymer complex with Liquid Fast Red (LV, TA-125-AL) substrate buffer and chromogen,
g) counterstaining with Mayer's haematoxylin (TA-125-MH).

For each test a positive control slide with the HepG2 carcinoma cell line was treated under the same conditions. Identification of epithelial (CK+) cells was performed using the Leitz Dialux 20 EB microscope connected to a Leica DC 300F camera.

### Example 1g: Cell culture

Human carcinoma cell lines HepG2 (liver), HCT116 (colon) and A549 (lung) were obtained from the American Type Tissue Collection (ATTC) and maintained in culture flasks at 37°C with 5% CO₂ in a humidified atmosphere. Cells were grown in Dulbecco's Minimum Essential Medium High Glucose with L-Glutamin (PAA) containing 10% FBS (Biochrom AG) and 1% gentamycine (CC-Pro). Media for HepG2 cell line was additionally supplemented with 1% sodium pyruvate (PAA) and 1% nonessential amino acids (PAA). Cells were detached from the bottle by trypsinising.

### Example 1h: Clinical characteristics of patients for CTC quantification & gene expression analysis.

Within this invention patients' blood was drawn consecutively during treatment visits in outpatient unit. The clinicopathological data of the patients used for CTC quantification in this pilot study is listed in Table 1. Clinicopathological data of patients used for identification of molecular markers out of the positively enriched EpCAM+ CTCs is shown in Table 2. All blood samples were collected before or during treatment in intervals of 7 days for the first 3-4 drawings and later in case of follow-up visits in our outpatient unit. In the expression analysis prove-of-principle study and validation group, blood was also accepted for testing when drawn shortly after SIRT respectively planned time-points.

### RNA extraction

Total RNA was extracted from recovered EpCAM-positive tumor cells and from human carcinoma cell lines using MagAttract RNA Cell Mini M48 Kits (Qiagen) and King Fisher mL magnetic particle processor (Themo Fisher) according to the manufacturer's protocol. Subsequently, remaining DNA was removed using RQ1 RNase free DNase (Promega) according to the instruction supplied.

### Real time RT-PCR

One-step real time RT-PCR (Roche LightCycler 480) was performed using Quanti Fast SYBR Green RT-PCR Kits and QuantiTect primer assays (Qiagen) for the following genes: TGFb1 (QT00000728), HGFAC (QT00000077), HIF1a (QT00083664), KRT19 (QT00081137), KRT18 (QT01846327) AFP (QT00085183), APC (QT00038962), IGFBP1 (QT00049427) and EpCAM (QT00000371). The primers for reference gene b-actin (Eurofins MWG) were as follows: forward: 5'-GAGCGCGGCTACAGCTT-3', reverse: 5'-TCCTTAATGTCACGCACGATTT-3'. Assays were performed in triplicates to determine expression levels. Thermal cycling conditions were 10 min at 50°C and 5 min at 95°C for RT and initial denaturation followed by 50 cycles of 95°C for 10 sec and 60°C for 30 sec. Triplicates of HepG2- HCT116- and A549-RNA (10ng/µl) were used as internal standard to control each run. Each primer has been validated in a serial dilution of RNA extracted from cell lines mentioned above.

### Example 11: Statistical Analysis

Spearman's rank correlation was used to examine associations between CTC subtypes and clinical characteristics of the patients. Associations of the cell type ratios and progression-free survival (PFS) were tested by the Kaplan-Meier method. Survival differences between patients with a high- and low-cell type ratio were analyzed by the log-rank test. For gene expression analyses Wilcoxon signed rank test was used to assess whether expression levels differ in CTC enriched and hematologic cell fractions.

The associations among gene expression levels and clinicopathologic parameters and between each gene expression were tested with Spearman test for bivariate correlations. To detect independent prognostic factors associated with progression each gene was tested in a linear backward multivariate regression model along with established clinical tumor markers AFP, Bilirubin and LDH with progression as dependent variable. Gene expressions that showed significant correlation to therapy response were then split in high- and low-level groups by setting a cut-point at the 50^{th} percentile Receiver operating characteristic (ROC) curve analysis was used to test the ability of the chosen cutoffs to discriminate progression from partial response (stable disease). The level of significance was set to P<0,05. All P values were based on two-sided tests. All statistical analyses were performed using the Software Packages SPSS for Windows (Version 19.0; SPSS, Inc, Chicago, IL) and Medcalc, Version 12.3.0 (Mariakerke, Belgium).

### Example 2: Results

### Immunocytochemical Identification of CTC subtypes

Objects that stained positive for DAPI and pan-cytokeratin, EpCAM or ASGPR1 and negative for CD45 were captured and considered as tumor cells. Pan-CK+/EpCAM+/CD45- as well as pan-CK+/EpCAM-/CD45- cells were detected after negative isolation. Furthermore pan-CK+ cells were detected in the remaining bead-free cell suspension after CD45 depletion and removal of EpCAM-positive CTC supporting the occurrence of a CK+/EpCAM- CTC subpopulation. Furthermore, cells with mesenchymal features such as N-Cadherin+/CD45- and Vimentin+/CD45- were detected after negative isolation. Cells showing both epithelial and mesenchymal characteristics such as N-Cadherin+/CK+/CD45- and vimentin+/CK+/CD45- were also found. Cells that stained positive for potential markers of circulating tumor cells and CD45 were also enumerated.

### Subtypes and clinical outcome

Spearman's rank test resulted in a significant association of progression free survival (PFS) with the ratio N-Cadherin+/CK+ (p=0.04) and the ratio vimentin+/CK+ (p=0.02). Both mesenchymal/epithelial CTC ratios were by trend significantly correlated to each other (p=0.07). Tumor size was significantly correlated to therapy response (p=0.03) but displayed a trend to significance in correlation to the ratio N-Cadherin+/CK+ (p=0.1). The amount of epithelial CK+ cells and Child status also correlated by trend towards significance (p=0.1). To test whether the N-Cadherin+/CK+ ratio or the Vimentin+/CK+ ratio potentially divide patients according to their chance of a prolonged PFS Kaplan-Meier Survival Analysis was used. The Kaplan-Meier log rank test showed that both ratios are significantly associated with PFS. A Vimentin+/CK+ ratio higher than 0.5, meaning that there were half as many Vimentin positive cells then CK+ cells was associated with a longer median PFS (2 months, p=0.01; [HR]=0.18). A N-Cadherin+/CK+ ratio lower than 0.1 resembled an association to shortened progression free survival (p=0.03; [HR]=0.19).

Identification of pan-cytokeratin-positive and Hep-Par-1-positive CTC after sequential positive enrichment.

The enrichment of epithelial cells using anti-EpCAM immunomagnetic beads was confirmed by immunocytochemical staining against pan-CK or Hep-Par-1, respectively. By microscopic observation, the EpCAM+ enriched (bead-bound) cell suspension contained epithelial cells that stained positive for pan-CK or Hep-Par-1 among hematopoietic cells. Due to shearing forces between beads, magnet and cells the membrane may sometimes rupture and cytoplasm leaks which leads to pink background staining. Results were reviewed by a clinical pathologist.

mRNA expression of EpCAM and KRT18 in depleted and enriched cell suspensions.

To confirm the depletion step using anti-CD45 coated immunomagnetic beads and the subsequent CTC enrichment step using anti-EpCAM coated beads the mRNA expression of epithelial genes EpCAM and KRT18 in the CD45+, EpCAM+ and the remaining bead free cell fractions was examined. Expression levels of the epithelial cell surface marker EpCAM and the cytoplasmic intermediate filament protein KRT18 were significantly (p=0.0137 resp. p=0.0295) higher in the bead-bound EpCAM+ CTC enriched fraction compared to the CD45+ selected cell suspension. The EpCam mRNA expression was decreased (p=0.1) in the CD45+ selected hematopoietic cell fraction compared to the remaining bead-free cell fraction. EpCAM mRNA expression was slightly decreased in the remaining bead-free cell fraction compared to the EpCAM+ CTC enriched suspension. Comparing the EpCam+ respectively CD45+ selected cell suspensions with the remaining cell suspension KRT18 mRNA expression was significantly elevated (p=0.009 resp p=0.0002).

PCR-based identification of potential markers of progression (SIRT).

The statistical associations of the abovementioned genes was tested, known clinicopathological parameters such as AFP, LDH and Bilirubin as well as treatment outcome with Spearman test. Progression was significantly correlated to serological tumor markers AFP (p= 0.04) and LDH (p= 0.003). Krt19 mRNA expression was significantly correlated to progression (p=0.05) and serological bilirubin (p=0.02, Spearman rank correlation coefficient: r=0.7). APC respectively AFP gene expressions were significantly correlated with bilirubin (p=0.05 r=-0.6 resp. p=0.03, r=0.6). Expression levels of Hifia mRNA (p=0.05, r=0.6) and HGFAC (p=0.04, r=0.6) were significantly correlated to serological AFP whereas IGFBP1 (p=0.03, r=-0.7) and TGFB (p=0.03, r=-0.6) mRNA expression significantly but inversely correlated with LDH. IGFBP1 mRNA expression was significantly associated to AFP (p=0.0065, r=0.9), APC (p=0.0217, r=0.7), HGFAC (p=0.0125, r=0.8) and KRT19 (p=0.0053, r=0.8) and showed by trend significant correlation to Hif1a (p=0.0667, r=0.6).

Additionally, the changes of mRNA expression levels between time points in patients with progression versus disease control were investigated. Gene expression levels of AFP, HGFAC, TGFb, Hif1a,and APC showed the largest changes between first and third, respectively second and third time point, whereas IGFBP1 mRNA expression showed the biggest differences between first and second time-point (p=0.06). Each gene was tested as a continuous variable in separate linear backward multivariate regression models along with established clinical tumor markers AFP, Bilirubin and LDH including progression as dependent variable. IGFBP1 mRNA expression showed the highest independent significant correlation to response (p=0.0062; model fit p=0.012) followed by HGFAC (p=0.0178; model fit p=0.008) and AFP (p=0.04; model fit p=0.008). TGFb and APC mRNA expressions showed tendency to significance at third time point (p= 0.1117; p= 0.1325)..AFP was excluded from the model when tested together with IGFBP1 mRNA expression (model fit p=0.01) .

Patients were than split into low and high expression groups with either progression or partial response (stable disease) by choosing the cut-points at the 50^{th} percentile. To test whether gene expression levels were significantly different in-between subgroups the linear backward multivariate regression models including AFP, Bilirubin and LDH was repeted. Low expression of IGBFP (p=0.01) and serological Bilirubin (p=0.02) were the only factors in this patient cohort showing a significant independent correlation to response. The overall model fit had a significance level of p=0.016 (<0.05). High AFP mRNA expression (p=0.0566) as well as serological AFP (p=0.0792) were by trend significant and Bilirubin (0.052) was significantly correlated to response (over-all model fit of p=0.011).

An independent set of patients with similar clinicopathological characteristics (Table 2) was used as a validation set and assessed the IGFBP1 mRNA expression following the procedure described above. Spearman's rank test confirmed significant correlations of IGFBP1 expression with serological tumor markers LDH (p=0.03) and Bilirubin (p=0.01). Again, a decrease of IGFBP1 expression during course of therapy was significantly associated with progression (p=0.0134; model fit p=0.015).

Kaplan-Meier log rank test was used to examine the association of the identified change in IGFBP1 expression between time-points and progression free survival. A decreased IGFBP1 expression significantly correlated to shortened PFS (p=0.04). These results were confirmed by Cox multivariate regression analysis (p=0.005; HR=0.02; data not shown due to low patient numbers) High IGFBP1 expression showed sensitivity (true positive rate) of 80% and specificity (true negative rate) of 80% for the diagnosis progression versus disease control. The area under the curve was 0.8 (CI 0.44 to 0.98) with a significance level of p = 0.03.

### Example 3: Circulating CD133+ stem and mesenchymal cells in peripheral blood of a patient with c-Met positive non-small cell lung cancer treated with Cisplatin/Pemetrexed (CisPem).

It has already been discussed that circulating tumor cells (CTC) may have potential as an easily accessible surrogate marker for therapeutic decisions in patients with non-small cell lung cancer. CD133 is expressed by stemcells and has been described as an indicator for shortened progression-free survival in Lung Cancer patients receiving Cisplatin treatment. N-Cadherin seems to be a marker for epithelial-mesenchymal-transition, a process that also may indicate limited response to therapy. In association to this c-Met is pictured as a stem cell marker. Furthermore c-Met plays a role in the epithelial-mesenchymal transition during cancer invasion.

Mononuclearic cells including CTCs were enriched using a Ficoll density gradient followed by depletion of hematopoietic cells employing a mixture of immunomagnetic beads linked with monoclonal CD45 and CD15 antibodies. The resulting cell suspension was subsequently spun on glass slides. Immunofluorescence double labeling with primary antibodies against CD45 was utilized to identify hematopoietic cells. Pan-CK, CD133 and N-Cadherin were taken as markers for circulating tumor cell subgroups. N-Cadherin and CD133+ were examined on different slides of the same patient at the same timepoint due to practical reasons. Furthemore cells were stained with DAPI to identify the nucleus. An average number of 2 CD133+ cells and 2 N-Cadherin+ cells plus 17 respectively 22 Pan-CK+ cells per 10 visual fields (vf) in 20x magnification on each slide (1360 vf/slide) was found.

In conclusion this example supports CTC subgroups as potential biomarkers of an individual therapeutic approach. The identified stem cells are in accordance with a c-Met overexpressing primary tumor. Many solid tumors consist of cells with different molecular characteristics. Certain cells like for instance stem cells or mesenchymal cells may distinctively be selected during the treatment of first choice and lead to relapse. An approach relying solely on the molecular characterization of the primary tumor upfront may be insufficient.

### Table 1: Patient demographics for quantification

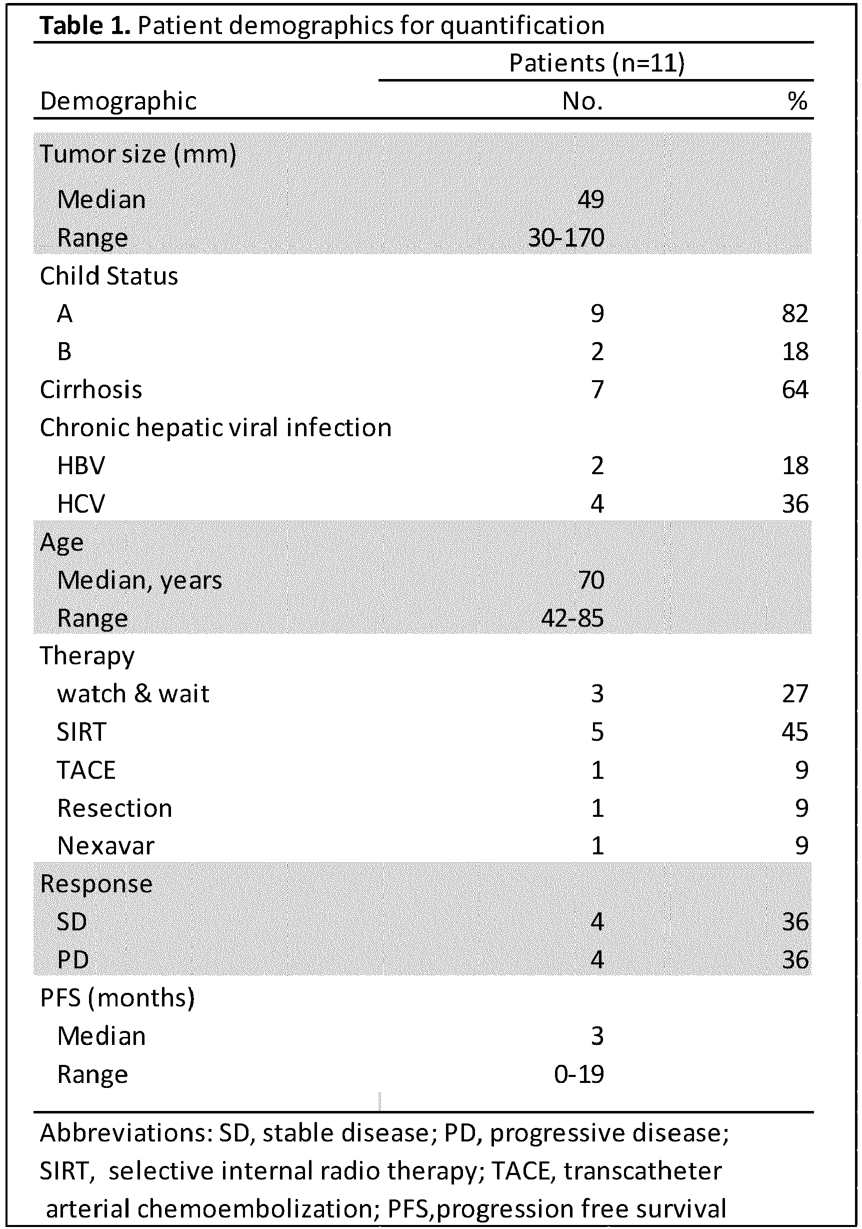

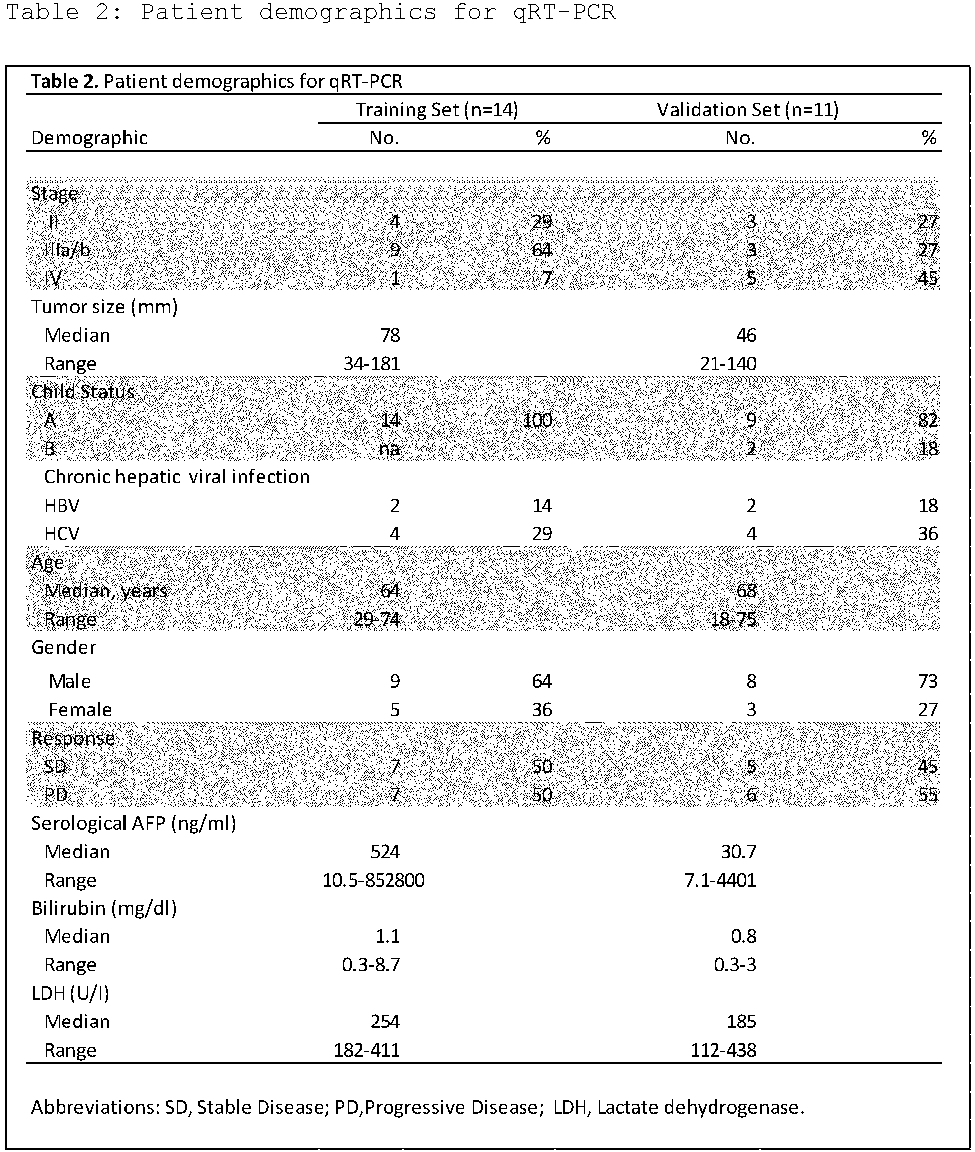

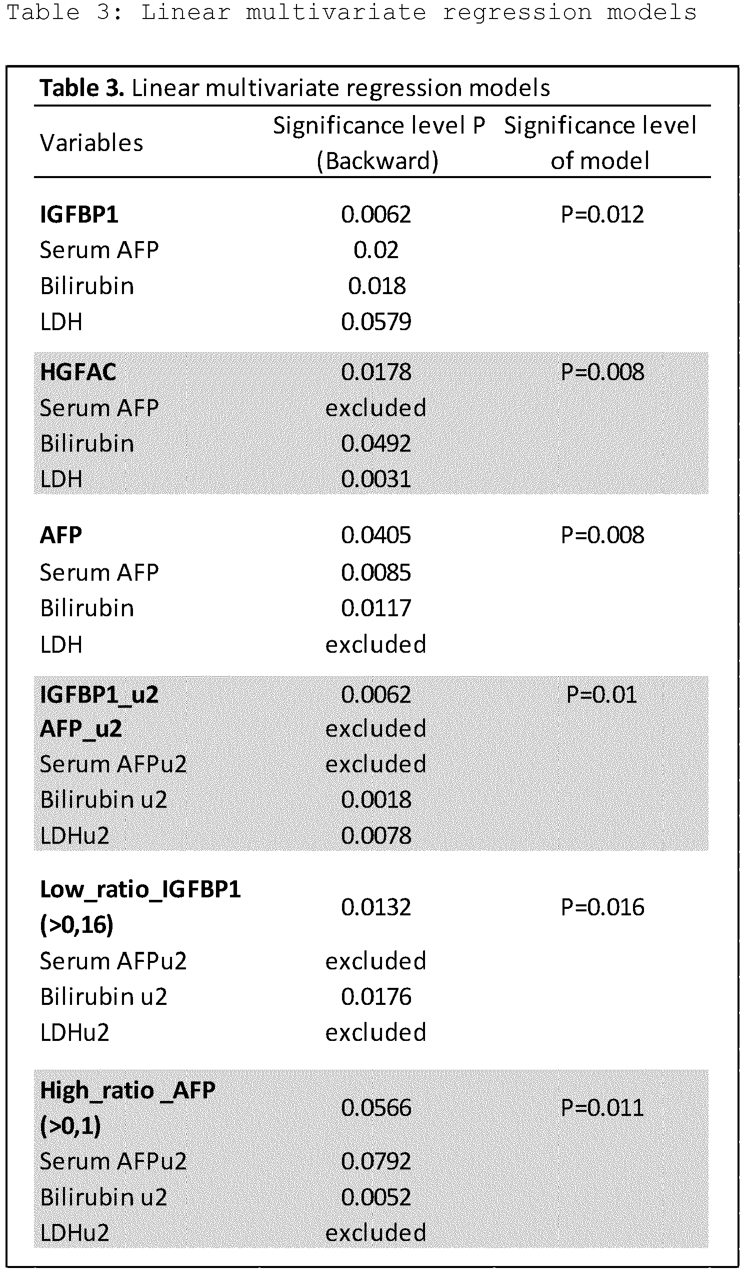

### Example 4: Circulating tumor cell composition and molecular markers in metastasized renal cell carcinoma (mRCC)

Individual CTC composition during first-line treatment in patients with mRCC was analyzed. Multi-immunofluorescence based morphological analysis revealed a variety of CTC subtypes with epithelial, mesenchymal, stem cell-like or mixed characteristics such as N-cadherin+/CK-/CD45-; N-cadherin-/CK+/CD45-; CD133+/CK+/CD45+ and CD133-/CK+/CD45 (low) cells. Analyses of individual CTC profiles indicated that the presence as well as the number of mesenchymal and stem cell-like CTC was associated to poor treatment response. The presence of stem cell-like CD133+ cells and the presence of mesenchymal N-cadherin+/CK- cells were correlated to a shortened PFS. If CD133+ cells were detectable, N-cadherin+/CK- cells were likely to be found. Analyses of individual CTC profiles indicated that the presence as well as the number of mesenchymal and stem cell-like CTC was associated to short progression-free survival. Elevated HIF1A, VEGFA, VEGFR and FGFR mRNA expression levels were associated with response to first-line anti-angiogenesis treatment and were inversely correlated to presence of cells with stem-cell or mesenchymal characteristics.

### Example 4.1: Preparation of blood samples and CTC enrichment

Duplicates of 20 ml citrated peripheral venous blood were drawn from mRCC patients about 4 months after baseline and processed within 24 h after collection. Blood sample preparation was done as follows: 20 ml of blood were diluted with 10 ml PBS and carefully layered into a Leucosep tube containing 16 ml Ficoll-Paque (GE-Healthcare) below a porous barrier. After bouyant density gradient centrifugation (1600 x g, 20 °C, 20 min) the interphase consisting of peripheral blood mononuclear cells (PBMNC) and CTC was removed and washed. We employed an enrichment strategy consisting of 2 steps. For (i) subtype analyses CTC were negatively enriched by hematopoietic cell depletion. PBMNC were treated with 50 µl of a 1:1 mixture of anti-CD45 and anti-CD15 coated immunomagnetic beads in a magnetic particle processor. The remaining cell suspension included bead-free pre-enriched tumor cells and was spun onto two glass slides per sample using the Cell Spin II centrifuge, air dried and subsequently fixated with 96% Ethanol. Slides were stored at 4°C until subjected to immunocytochemical staining. For (ii) gene expression analysis a second sample was prepared in the same manner, but epithelial CTC were further enriched using anti-EpCAM immunomagnetic beads (Epithelial Enrich, Dynabeads, Life Technologies, Carlsbad, USA) resulting in an EpCAM-positive CTC suspension for molecular analysis.

### Example 4.2: Identification of CTC subtypes using multi-fluorescence labeling.

Immunofluorescence staining of epithelial, mesenchymal, stem cell-like and hematopoietic cells was carried out in the CD45-depleted pre-enriched tumor cell suspension as described previously (Nel et al, 2013). Either pan-CK guinea pig polyclonal antibody and N-cadherin (EPR1792Y) rabbit monoclonal antibody (2019-1, Epitomics) or CD133 rabbit polyclonal antibody (orb18124, biorbyt) for CTC and anti-CD45 (MEM-28) mouse monoclonal antibody (ab8216, Abeam) for hematologic cells overnight at 4°C, wash in 0,1% Tween, binding of secondary antibodies (FITC-conjugated AffiniPure goat anti-rabbit and Cy3-conjugated AffiniPure goat anti-mouse or AlexaFlour647-conjugated AffiniPure F(ab')2 Fragment goat anti-guinea pig. As described previously, for each test a control slide with a mixture of PBMNC (CD45-positive, pan-CK-negative) from a healthy donor spiked with epithelial cells from the hepatocellular carcinoma cell line Hep G2 (CD45-negative, pan-CK-positive) was treated under the same conditions. Gastrointestinal stromal cells (GIST 882) were used as positive control for the mesenchymal marker N-cadherin. Stable transfected CD133-expressing K265 cells were used as positive control for stem cell marker CD133. Microscopic evaluation was carried out. Stained slides were manually examined and CTC were detected within the same areas, each consisting of 10 visual fields using a 20x magnification on both slides. Samples from 5 healthy donors were processed and examined under the same conditions in order to define cut-off values for false-positive events.

### Example 4.3: RNA extraction and qRT-PCR

For gene expression analyses epithelial CTC were sequentially enriched by depletion of hematopoietic cells and subsequent positive selection using anti-EpCAM immunomagnetic beads as described above. Total RNA was extracted from recovered EpCAM-positive tumor cells using MagAttract RNA Cell Mini M48 Kits (Qiagen, Hilden, Germany). Additionally, remaining DNA was removed using RQ1 RNase free DNase. One-step real time RT-PCR was performed for gene expression analysis of VEGFA; VEGFR (VEGFR); FGF2; FGFR; PDGFA and HIF1A.

### Example 4.4: Statistical Analysis.

Statistical tests were performed according to previously published studies by our group (Hoffmann et al, 2009; Hoffmann et al, 2010; Nel et al, 2013). The level of significance was set to P<0.05. All P values were based on two-sided tests. All statistical analyses were performed using the Software Packages JMP 10.0 Software (SAS, Cary, NC, USA), SPSS for Windows (Version 19.0; SPSS Inc., Chicago, IL) and Medcalc, Version 12.3.0 (Mariakerke, Belgium).

### Example 4.5: Results

### Immunofluorescence based identification of CTC subtypes.

For the investigation of cellular subtypes a multi-staining method was required in order to detect various epithelial, mesenchymal, stem cell-like and hematopoietic characteristics. Therefore, we used multi-fluorescence staining for CTC-subtype detection in mRCC patients. Stable transfected hematopoietic K562 cells and GIST 882 cells were used as controls for stem cell (CD133) and mesenchymal (N-cadherin) markers, respectively. In a mixture of Hep G2 cells and PBMNC hematopoietic cells displayed positive staining against CD45 while Hep G2 cells were CD45-negative. Hep G2 cells stained positive against the epithelial markers pan-CK and EpCAM. Objects that showed a positive nuclear staining with DAPI a negative staining against CD45 and a positive staining for pan-CK, N-cadherin or CD133 were captured and considered as tumor cells. In mRCC blood samples we detected cells with mesenchymal features such as N-cadherin+/CK-/CD45- and cells with epithelial properties like CK+/N-cadherin-/CD45- and cells with both characteristics like CK+/N-cadherin+. Also cells were detected showing stem cell-like features such as CD133+/ CK+ cells. In addition, cells were found that stained positive for potential markers of CTC and CD45 such as CK+/ CD45+ as well as CK+/ CD133+/ CD45+ cells. We also found a sub-population of cells staining positive for CK and CD45 (low) but negative for EpCAM and cells staining triple positive for CK, EpCAM and CD45 (low). CTC were enumerated and profiles of each patient were examined. The total amount of N-cadherin-positive, CK-positive and CD133-positive cells was summarized and a ratio of mesenchymal to epithelial cells was conceived after negative enrichment using CD45-depletion. The normalized and enumerated potential CTC against the total PBMNC number detected in the DAPI channel in each visual field and expressed the number of CTC per 1000 PBMNC. Analysis of samples from healthy donors revealed the following cut-off values for false positive events per 1000 PBMNC after CD45 depletion: 0.03 CD133+/CK+ cells; 0.04 CD133/CK-cells; 0.03 N-cadherin+/CK+ cells; 0.05 N-cadherin+/CK- cells and a total of 0.6 CK+ cells.

### CTC-subtypes and clinical outcome.

It was found that the number of mesenchymal N-cadherin+/CK-cells showed the strongest association to response and progression free survival (PFS). The second strongest factor was the number of CD133+ cells. Spearman's rank correlation revealed a significant association of N-cadherin+/CK- to the presence of CD133+ cells (p=0.03; r=0.73) as well as to the number of CD133+/CK- cells/1000 PBMNC (p=0.03; r=0.71) and confirmed a significant correlation to response (p=0.05; r=0.43) and PFS (p=0.04; r=-0.59). Furthermore, the number of N-cadherin+/CK- cells was correlated to tumor staging (p=0.04; r=0.63) and serological creatinine level (p=0.04; r=-0.83). Mann-Whitney test showed that the number of N-cadherin+/CK-was significantly lower in responders vs. non-responders (p=0.049). ROC curve was used for analysis to assess the sensitivity and the specificity of N-cadherin+/CK- cells at the cut-point 50th percentile (0.45) to distinguish stable disease from partial response. The area under the curve was 0.8 (CI 0.49 to 0.96) with a significance level of p=0.0002. Kaplan-Meier test showed that an increased number of N-cadherin+/CK- cells (>0.35) significantly correlated to shortened PFS (8.5 vs. 15 months; p=0.04; [HR]=0.36).

Partition tree analysis revealed that the number of CD133+ cells was the second strongest divisor and the cut-point was defined at 0.23 cells/ 1000 PBMNC after enrichment. Mann-Whitney test revealed that PFS during first-line treatment was significantly and nearly a year shorter when CD133+ cells were present (p=0.04; 8 vs. 18 months). Kaplan-Meier test confirmed shortened PFS in the presence of CD133+ cells (p=0.004; 8 vs. 18 month [HR] =3.6). The ratio of N-cadherin+ cells to CK+ cells (mesenchymal to epithelial cell type) increased significantly when CD133+ cells were present (p=0.02). A significantly increased number of N-cadherin+ cells was detected in the presence of CD133+ cells (p=0.02) and in turn a significantly increased number of CD133+ cells in the presence of N-cadherin+ cells (p=0.05). Kaplan-Meier test confirmed a significantly shortened PFS when N-cadherin+ cells were present (p=0.05; 8.5 vs. 17 months; [HR]=3.1)

The presence of CD133+ cells along with established clinical markers (LDH, urea, creatinine) was included in a backward linear multivariate regression model. The overall model fit had a significance level of p=0.03 (<0.05). The presence of CD133+ cells (p=0.02), creatinine (p=0.03) and LDH (p=0.03) showed a significant independent correlation to response.

Gene expression analysis in enriched (EpCAM+) and depleted (CD45-/EpCAM-) cell fractions.

Gene expression levels of candidate genes were measured in the EpCAM-enriched (EpCAM+) and in the remaining CD45- and EpCAM-depleted (CD45-/EpCAM-) fraction containing cells that are EpCAM-negative potentially since they underwent EMT or because they are not of epithelial origin. Wilcoxon test showed that the FGFR mRNA expression was significantly increased in the remaining CD45-/EpCAM- fraction compared to the EpCAM+ fraction (p=0.03). VEGFA expression was significantly increased in the EpCAM+ fraction compared to the remaining CD45-/EpCAM- fraction (p=0.004).

### Gene expression analyses and clinical factors

Partition tree analyses based on PFS as dependent variable and found HIF1A mRNA expression in the EpCAM+ fraction was found to be the strongest divisor of this patient cohort with the cut-point at the 50^{th} percentile. Kaplan-Meier test showed that an increase in HIF1A mRNA expression was by trend significantly associated with shortened PFS (p=0.1).

FGFR mRNA expression in the remaining CD45-/EpCAM- fraction was the second strongest divisor of the patient cohort while significantly increased in responders (p=0.01). ROC curve analysis of FGFR mRNA expression (>60^{th} percentile; >0.94) revealed a specificity of 83% and a sensitivity of 70% for predicting response. The area under the curve was 0.8 (CI 0.49 - 0.94) with a significance level of p=0.02.

### Expression levels of HIF1A (CD45-/EpCAM-, p=0.05, r=0,56), PDGFA (CD45-/EpCAM-, p=0.01, r=0.67) and VEGFR (CD45-/EpCAM-, p=0.01, r=0.71) were significantly correlated to LDH. The test indicated a significant association of response with FGFR gene expression (p=0,001; r=0,72).

Statistical associations among gene expression levels were analyzed separately in the depleted CD45-/EpCAM- and the enriched EpCAM+ fraction. VEGFA was significantly associated to FGFR (p=0.03) in the EpCAM+ fraction, to HIF1A (p=0.03) in both, the CD45-/EpCAM- (p=0.02) and EpCAM+ (p=0.03) fractions, to VEGFR in both, the CD45-/EpCAM- (p=0.02) and EpCAM+ (p=0.0001) fractions and to PDGFA in both, the CD45-/EpCAM-(p=0.04) and EpCAM+ (p=0.001) fractions. VEGFA mRNA expression in the CD45-/EpCAM- fraction was significantly correlated to FGF2 (p=0.04) in the EpCAM+ fraction. VEGFR was significantly associated with FGFR in the EpCAM+ fraction (p=0.05), to PDGFA in both, the CD45-/EpCAM- (p=0.04) and EpCAM+ (p=0.001) fractions (p=0.04), to HIF1A in the CD45-/EpCAM- fraction (p=0.002) and showed a by trend significant correlation to HIF1A (p=0.06) and to FGF2 (p=0.07) in the EpCAM+ fractions. HIF1A was significantly correlated to PDGFA in the CD45-/EpCAM- fraction (p=0.001) and showed a trend towards significant correlation to FGFR in the EpCAM+ fraction (p=0.08). FGFR was significantly associated to FGF2 in the CD45-/EpCAM- fraction (p=0.0002). PDGFA was by trend significantly correlated to FGF2 in both, the CD45-/EpCAM-(p=0.06) and the EpCAM+ fraction (p=0.07)

Patients were then split into low and high expression groups with the cut-point chosen at the 50^{th} percentile. Each gene expression was separately tested in a linear multivariate regression model along with established clinical markers (LDH, uric acid, creatinine, calcium) and response as independent variable. In the EpCAM+ fraction the following gene expressions had a significant correlation to response and lead to regression models with a significant overall model fit: HIF1A (p=0.02; overall model fit: p=0.03), VEGFR (p=0.06; overall model fit: p=0.001), VEGFA (p=0.02; overall model fit: p=0.001), PDGFA (p=0.07; overall model fit: p=0.001). In the remaining CD45-/EpCAM- depleted fraction expression levels of the following genes were significantly associated to response and revealed significant multivariate regression models: VEGFR (p=0.026; overall model fit: p=0.068); VEGFA (p=0.001; overall model fit: p=0.005); FGFR (p=0.04; only factor remaining in the model). Furthermore, the abovementioned gene expressions were tested together along with clinical markers in a multivariate linear regression model with response as dependent variable. In the EpCAM+ fraction VEGFR expression was the only factor in the model showing a significant independent correlation to response (p=0.03) while HIF1A, VEGFA, PDGFA, creatinine, uric acid and calcium were excluded. In the EpCAM-/CD45- fraction the only factor retaining in the model was FGFR gene expression while VEGFR, VEGFA, creatinine, uric acid and calcium were excluded.

### Gene expression and CTC composition

Mann-Whitney test showed that HIF1A and VEGFR mRNA expression levels were significantly decreased (p=0.05) in the EpCAM-/CD45-depleted fraction when CD133+ cells were present in the blood sample. A by trend significant decrease of VEGFA mRNA expression was observed when CD133+ cells were present (p=0.12). To combine morphological cell type detection with gene expression analysis we included the presence of CD133+ cells and FGF2 gene expression along with established clinical markers (LDH, creatinine, urea) in a backward linear multivariate regression model with response as dependent variable. The overall model fit had a significance level of p=0.03 and all factors showed significantly independent correlation to response: FGF2 gene expression (p=0.01), CD133 presence (p=0.02), LDH (p=0.03), urea (p=0.03) and creatinine (p=0.05).

### Example 5: Formation and repair kinetics of Pt-(GpG) DNA adducts in extracted circulating tumour cells and response to platinum treatment

Pt-(GpG) intrastrand crosslinks are the major DNA adducts induced by platinum-based anticancer drugs. In the cell lines and mouse models, the persistence of these lesions correlates significantly with cell damage. Pt-(GpG) DNA adducts in CTCs treated with cisplatin in medium upfront to systemic therapy from patients with advanced non-small-cell lung cancer (NSCLC) was studied.

Blood was drawn before systemic treatment and the CD45/CD15-depleted fraction of mononuclear cells was exposed to cisplatin, verified for the presence of CTC by pan-cytokeratin (pCK) staining and immunoanalysed for the level of Pt-(GpG) in DNA. Pt-(GpG) adducts can be detected in CTC from NSCLC patients and assessing their kinetics constitutes a clinically feasible biomarker for response prediction and dose individualisation of platinum-based chemotherapy.

### Example 5.1: Immunocytochemical identification of CTC and measurement of Pt-(GpG) adducts.

Epithelial CTCs were detected that stained positive for DAPI and pCK and negative for CD45. The ratio of pCK-positive to CD45-positive cells in the depleted fractions was in the range of p1-5000, resulting in up to three CTC per cell spot on the slides. CD45-positive cells depicted nearly 10-fold lower levels of DNA platination after exposure to cisplatin as compared with CTC. The same intercellular distribution was observed in all the cell samples, including those isolated under systemic therapy with cisplatin.

### Example 5.2: Pt-(GpG) kinetics in CTC exposed to cisplatin ex vivo.

The measurement of Pt-(GpG) adducts in the nuclear DNA of short term-exposed CTC depict a characteristic time course of formation and removal. Adduct kinetics were reproducible when repeated in individual blood samples from the same donor but showed broad variations between CTC from individual patients. Formation and persistence of DNA platination can be characterised by different parameters such as curve inclination between distinct time points (slope), peak adduct level (AFUmax) and area under the curve (AUC). The degree of association was calculated between these three values of ex vivo adduct persistence in CTC from 11 patients with their therapeutic response using the Spearman's rho rank correlation coefficient. AFUmax correlated significantly with AUC (P.0.001) and response (P.0.05). The AUC was also significantly correlated with response (P.0.03). A linear backward multivariate regression model with response as the dependent variable identified AUC as the only factor significantly and independently associated with response (P.0.002). Therefore, summary measures of ex vivo Pt-(GpG) adduct levels from patients before treatment were expressed in AUC. Persistence data did not show a normal distribution, and a Mann-Whitney test was performed for a non-parametric test, which revealed a significant difference between patients showing stable disease or partial response 6 weeks after systemic platinum-based treatment (P.0.01; n.11). Recursive descent partition tree analysis was used to find the factor showing the strongest association with response and define the optimal cut-point of this factor. Response vs non-response was used as the defining factor and adduct persistence values.

### Example 5.3: (AFUmax, AUC, slope) alongside ERCC1 mRNA expression and clinical factors such as age, histology, staging (TNM) and grading as dividing factors.

The strongest divisor in this study group was AUC followed by AFUmax. The most significant split determined by the largest likelihood-ratio chi-square statistic of AUC was the 50th percentile (4.166). According to the descent recursive partition analysis, patients were then split into low and high AUC groups at the 50th percentile. A stepwise linear multivariate regression analysis using AUC as a dichotomous variable confirmed high AUC (450th percentile) as being independently associated with response with an overall model fit of P.0.036. Patients with a high AUC, and therefore higher ex vivo adduct persistence in tumour cells incubated with cisplatin upfront to intravenous therapy, had a higher chance to benefit from systemic cisplatin treatment. Receiver operating curve (ROC) analysis was used to assess the sensitivity and specificity of high AUC (450th percentile) as a parameter of ex vivo Pt-(GpG) persistence to predict response from patients upfront to cytotoxic therapy and revealed a sensitivity of 80% and a specificity of 83% with a significance of P.0.01 and an ROC area under the curve of 0.817.

To test whether adduct kinetics measured in tumour cells after ex vivo exposure to cisplatin mirror the formation and repair rates for Pt-(GpG) in the DNA of those cells in vivo during systemic treatment, blood samples from the same patient were analysed under both the conditions. Specimen drawn at baseline and 2, 4 and 24 h after cisplatin infusion exhibited similarly shaped courses as measured in CTC aliquots ex vivo. In this case, the clinical dose of cisplatin (50 mgm_2) translating into 1.4 mg kg_1 body weight resulted in CTC adduct levels comparable to a short time ex vivo exposure with 20 mgml_1 for 2 h.

### Example 5.4: Adduct persistence and ERCC1 mRNA expression.

For ERCC1 gene expression analysis, we used a sequential enrichment strategy to physically isolate tumour cells from PBMNC. After depletion of white blood cells with anti-CD45 and -CD15 immunomagnetic beads, EpCAM+ cells were enriched from the resulting cell suspension for downstream analysis. ERCC1 mRNA levels were measured in selected CTC isolated upfront to cytotoxic chemotherapy. The RT-PCR-based data showed no association with ex vivo Pt-(GpG) adduct persistence values, including slope, AFUmax and AUC (P.0.67, P.0.44 and P.0.78, respectively). Although the mean expression ratio of ERCC1 was nearly twofold higher (1.02±0.58) in the patient group with stable disease as compared with patients with partial response (0.69±0.23), no significant correlation with response was observed (P.0.55).

### Literature:

1. Llovet JM, Burroughs A, Bruix J. Hepatocellular carcinoma. Lancet 2003;362:1907-1917.
2. Jemal A, Bray F, Center MM, Ferlay J, Ward E, Forman D. Global cancer statistics. CA Cancer J Clin 2011;61:69-90.
3. Belghiti J, Panis Y, Farges O, Benhamou JP, Fekete F. Intrahepatic recurrence after resection of hepatocellular carcinoma complicating cirrhosis. Ann Surg 1991;214:114-117.
4. Bruix J, Sherman M. Management of hepatocellular carcinoma. Hepatology 2005;42:1208-1236.
5. Lai Q, Avolio AW, Lerut J, Singh G, Chan SC, Berloco PB, Tisone G, et al. Recurrence of Hepatocellular Cancer after Liver Transplantation: The Role of Primary Resection and of Salvage Transplantation in East and West. J Hepatol 2012.
6. Toso C, Cader S, Mentha-Dugerdil A, Meeberg G, Majno P, Morard I, Giostra E, et al. Factors predicting survival after post-transplant hepatocellular carcinoma recurrence. J Hepatobiliary Pancreat Sci 2012.
7. Sangro B, Bilbao JI, Boan J, Martinez-Cuesta A, Benito A, Rodriguez J, Panizo A, et al. Radioembolization using 90Y-resin microspheres for patients with advanced hepatocellular carcinoma. Int J Radiat Oncol Biol Phys 2006;66:792-800.
8. Lau WY, Lai EC, Leung TW. Current role of selective internal irradiation with yttrium-90 microspheres in the management of hepatocellular carcinoma: a systematic review. Int J Radiat Oncol Biol Phys 2011;81:460-467.
9. Sun YF, Yang XR, Zhou J, Qiu SJ, Fan J, Xu Y. Circulating tumor cells: advances in detection methods, biological issues, and clinical relevance. J Cancer Res Clin Oncol 2011;137:1151-1173.
10. Lianidou ES, Markou A. Circulating tumor cells in breast cancer: detection systems, molecular characterization, and future challenges. Clin Chem 2011;57:1242-1255.
11. Hou JM, Krebs M, Ward T, Sloane R, Priest L, Hughes A, Clack G, et al. Circulating tumor cells as a window on metastasis biology in lung cancer. Am J Pathol 2011;178:989-996.
12. Krebs MG, Sloane R, Priest L, Lancashire L, Hou JM, Greystoke A, Ward TH, et al. Evaluation and prognostic significance of circulating tumor cells in patients with non-small-cell lung cancer. Journal of clinical oncology : official journal of the American Society of Clinical Oncology 2011;29:1556-1563.
13. Waguri N, Suda T, Nomoto M, Kawai H, Mita Y, Kuroiwa T, Igarashi M, et al. Sensitive and specific detection of circulating cancer cells in patients with hepatocellular carcinoma; detection of human telomerase reverse transcriptase messenger RNA after immunomagnetic separation. Clin Cancer Res 2003;9:3004-3011.
14. Xu W, Cao L, Chen L, Li J, Zhang XF, Qian HH, Kang XY, et al. Isolation of circulating tumor cells in patients with hepatocellular carcinoma using a novel cell separation strategy. Clin Cancer Res 2011;17:3783-3793.
15. Vona G, Estepa L, Beroud C, Damotte D, Capron F, Nalpas B, Mineur A, et al. Impact of cytomorphological detection of circulating tumor cells in patients with liver cancer. Hepatology 2004;39:792-797.
16. Guo J, Yao F, Lou Y, Xu C, Xiao B, Zhou W, Chen J, et al. Detecting carcinoma cells in peripheral blood of patients with hepatocellular carcinoma by immunomagnetic beads and rt-PCR. J Clin Gastroenterol 2007;41:783-788.
17. Schulze K, Beneken C, Staufer K, Nashan B, Lohse AW, Pantel K, Riethdorf S, et al. Detection of Epcam-Positive Circulating Tumor Cells in Patients with Hepatocellular Carcinoma - a Pilot Study. Hepatology 2011;54:1357a-1357a.
18. Moldenhauer G, Momburg F, Moller P, Schwartz R, Hammerling GJ. Epithelium-specific surface glycoprotein of Mr 34,000 is a widely distributed human carcinoma marker. Br J Cancer 1987;56:714-721.
19. Cristofanilli M, Budd GT, Ellis MJ, Stopeck A, Matera J, Miller MC, Reuben JM, et al. Circulating tumor cells, disease progression, and survival in metastatic breast cancer. N Engl J Med 2004;351:781-791.
20. Maheswaran S, Sequist LV, Nagrath S, Ulkus L, Brannigan B, Collura CV, Inserra E, et al. Detection of mutations in EGFR in circulating lung-cancer cells. N Engl J Med 2008;359:366-377.
21. Allard WJ, Matera J, Miller MC, Repollet M, Connelly MC, Rao C, Tibbe AG, et al. Tumor cells circulate in the peripheral blood of all major carcinomas but not in healthy subjects or patients with nonmalignant diseases. Clin Cancer Res 2004;10:6897-6904.
22. Paterlini-Brechot P, Benali NL. Circulating tumor cells (CTC) detection: clinical impact and future directions. Cancer Lett 2007;253:180-204.
23. Kalluri R, Weinberg RA. The basics of epithelial-mesenchymal transition. J Clin Invest 2009;119:1420-1428.
24. Yu M, Stott S, Toner M, Maheswaran S, Haber DA. Circulating tumor cells: approaches to isolation and characterization. J Cell Biol 2011;192:373-382.
25. Gradilone A, Raimondi C, Nicolazzo C, Petracca A, Gandini O, Vincenzi B, Naso G, et al. Circulating tumour cells lacking cytokeratin in breast cancer: the importance of being mesenchymal. J Cell Mol Med 2011;15:1066-1070.
26. Raimondi C, Gradilone A, Naso G, Vincenzi B, Petracca A, Nicolazzo C, Palazzo A, et al. Epithelial-mesenchymal transition and sternness features in circulating tumor cells from breast cancer patients. Breast Cancer Res Treat 2011;130:449-455.
27. Joly E, Hudrisier D. What is trogocytosis and what is its purpose? Nat Immunol 2003;4:815.
28. Marrinucci D, Bethel K, Bruce RH, Curry DN, Hsieh B, Humphrey M, Krivacic RT, et al. Case study of the morphologic variation of circulating tumor cells. Hum Pathol 2007;38:514-519.
29. Hofman V, Ilie MI, Long E, Selva E, Bonnetaud C, Molina T, Venissac N, et al. Detection of circulating tumor cells as a prognostic factor in patients undergoing radical surgery for non-small-cell lung carcinoma: comparison of the efficacy of the CellSearch Assay and the isolation by size of epithelial tumor cell method. Int J Cancer 2011;129:1651-1660.
30. Zhou L, Liu J, Luo F. Serum tumor markers for detection of hepatocellular carcinoma. World Journal of Gastroenterology 2006;12:1175-1181.
31. Gong Y, Cui L, Minuk GY. The expression of insulin-like growth factor binding proteins in human hepatocellular carcinoma. Mol Cell Biochem 2000;207:101-104.

## Claims

1. Method for analyzing the composition of circulating tumor cell (CTC) population in a biological sample from an individual for use in the diagnosis or prognosis of a tumor comprising the steps
a) isolation or enrichment of peripheral blood mononuclear cells (PMBCs) and CTCs from the biological sample,
b) depleting hematopoietic cells from the isolated or enriched PMBCs and CTCs,
c) testing the obtained cell suspension from step b) for at least two markers independently from each other selected from
epithel markers, wherein the epithel markers are selected from the group comprising epithelial cell adhesion molecule (EpCAM), epithelial growth factor receptor (EGFR), cytokeratin (CK), pan-cytokeratin (Pan-CK), E-Cadherin,
and/or
mesenchymal markers, wherein the mesenchymal markers are selected from the group comprising N-Cadherin and Vimentin,
and/or
tumor markers, wherein the tumor markers are selected from the group comprising AFP, KRT18, KRT19, TGFb, HIF1a, IGFBP1, IGFBP5, IGF, HGFAC, APC, VEGFA, PDGFA, FGFA, HIF1A, KDR1 (VEGFR), FGF2, HBsAg, ASGPR1, aSMA, Hep-Par-1,
and/or
markers for resistance namely C-Met,
and/or
stem cell markers namely CD133
and/or
hematopoietic markers, wherein the hematopoietic marker is selected from the group comprising CD45 or CD15,
and
d) thereby identifying one or more subgroup(s) of CTCs in the CTC population of that biological sample, wherein the composition of subgroup(s) of CTCs is characteristic for a specific tumor, wherein the tumor is selected from the group of non-small cell lung cancer (NSCLC), metastatic renal cell carcinoma (mRCC).

2. Method according to claim 1 comprising the step of isolating or enrichment of PBMCs and CTCs from the biological sample prior to depletion of hematopoietic cells.

3. Method according to claim 1 or 2 comprising the step of testing the obtained cell suspension for at least one additional marker selected from mesenchymal markers, epithel-to-mesenchymal transition (EMT) markers, markers for resistance namely C-Met, stem cell markers, hematopoietic markers.

4. Method according to one of the previous claims, wherein PMBCs and CTCs are isolated or enriched by density gradient centrifugation.

5. Method according to one of the previous claims, wherein hematopoietic cells are depleted using anti-CD45 and/or anti-CD15 antibodies, for example immunomagnetic beads coated with anti-CD45 and/or anti-CD15 antibodies.

6. Method according to one of the previous claims comprising the step of exposing the biological sample, the isolated or enriched PBMCs and CTCs or the cell suspension obtained after hematopoietic cell depletion to a pharmaceutical composition or a pharmaceutical compound.

7. Method according to one of the previous claims wherein the presence of the marker(s) in the CTC population is/are detected by immunofluorescence, PCR or FISH.

## Patentansprüche

1. Verfahren zum Analysieren der Zusammensetzung einer Population zirkulierender Tumorzellen (CTCs) in einer biologischen Probe eines Individuums, zur Verwendung bei der Diagnose oder Prognose eines Tumors, die folgenden Schritte umfassend:
a) Isolieren oder Anreichern von mononukleären peripheren Blutzellen (PMBCs) und CTCs aus der biologischen Probe,
b) Abreichern von hämatopoetischen Zellen aus den isolierten oder angereicherten PMBCs und CTCs,
c) Testen der erhaltenen Zellsuspension aus Schritt b) auf mindestens zwei Marker, die unabhängig voneinander ausgewählt werden aus
Epithelmarkern, wobei die Epithelmarker ausgewählt sind aus der Gruppe umfassend Epithel-Zelladhäsionsmolekül (EpCAM), Epithel-Wachstumsfaktorrezeptor (EGFR), Cytokeratin (CK), pan-Cytokeratin (pan-CK), E-Cadherin, und/oder
mesenchymalen Markern, wobei die mesenchymalen Marker ausgewählt sind aus der Gruppe umfassend N-Cadherin und Vimentin,
und/oder
Tumormarkern, wobei die Tumormarker ausgewählt sind aus der Gruppe umfassend AFP, KRT18, KRT19, TGFb, HIFla, IGFBP1, IGFBP5, IGF, HGFAC, APC, VEGFA, PDGFA, FGFA, HIF1A, KDR1 (VEGFR), FGF2, HBsAg, ASGPR1, aSMA, Hep-Par-1,
und/oder
Resistenzmarkern, als da wäre C-Met,
und/oder
Stammzellenmarkern, als da wäre D133,
und/oder
hämatopoetischen Markern, wobei der hämatopoetische Marker ausgewählt ist aus der Gruppe umfassend CD45 oder CD15,
und
d) dadurch Identifizieren einer oder mehrerer CTCs-Untergruppen in der CTC-Population dieser biologischen Probe, wobei die Zusammensetzung einer (oder mehrerer) CTC-Untergruppe(n) kennzeichnend ist für einen spezifischen Tumor, wobei der Tumor ausgewählt ist aus der Gruppe von nicht-kleinzelligem Lungenkrebs (NSCLC), metastatischem Nierenzellkarzinom (mRCC).

2. Verfahren nach Anspruch 1, den Schritt des Isolierens oder Anreicherns von PBMCs und CTCs aus der biologischen Probe vor der Abreicherung von hämatopoetischen Zellen umfassend.

3. Verfahren nach Anspruch 1 oder 2, den Schritt des Testens der erhaltenen Zellsuspension auf mindestens einen zusätzlichen Marker umfassend, der ausgewählt wird aus mesenchymalen Markern, Markern für einen Übergang von Epithel auf Mesenchym (EMT), Resistenzmarkern, als da wäre C-Met, Stammzellenmarkern, hämatopoetischen Markern.

4. Verfahren nach einem der vorangehenden Ansprüche, wobei PMBCs und CTCs durch Dichtegradientenzentrifugation isoliert oder angereichert werden.

5. Verfahren nach einem der vorangehenden Ansprüche, wobei hämatopoetische Zellen unter Verwendung von Anti-CD45- und/oder Anti-CD15-Antikörpern, zum Beispiel von mit Anti-CD45- und/oder Anti-CD15-Antikörpern beschichteten immunomagnetischen Beads, abgereichert werden.

6. Verfahren nach einem der vorangehenden Ansprüche, den Schritt des Einwirkenlassens einer pharmazeutischen Zusammensetzung oder einer pharmazeutischen Verbindung auf die isolierten oder angereicherten PBMCs und CTCs oder auf die nach der Abreicherung von hämatopoetischen Zellen erhaltene Zellsuspension umfassend.

7. Verfahren nach einem der vorangehenden Ansprüche, wobei das Vorhandensein des Markers (der Marker) in der CTC-Population durch Immunofluoreszenz, PCR oder FISH nachgewiesen wird.

## Revendications

1. Procédé d'analyse de la composition d'une population de cellules tumorales en circulation (CTC) dans un échantillon biologique provenant d'un individu pour une utilisation dans le diagnostic ou le pronostic d'une tumeur comprenant les étapes
a) d'isolement ou d'enrichissement en cellules mononucléées du sang périphérique (PMBC) et en CTC provenant de l'échantillon biologique,
b) d'appauvrissement en cellules hématopoïétiques provenant des PMBC et des CTC isolées ou enrichies,
c) de test de la suspension de cellules obtenue dans l'étape b) pour au moins deux marqueurs choisis chacun indépendamment de l'autre parmi
les marqueurs épithéliaux, dans lequel les marqueurs épithéliaux sont choisis dans le groupe comprenant la molécule d'adhérence cellulaire épithéliale (EpCAM), le récepteur de facteur de croissance épithéliale (EGFR), la cytokératine (CK), la pan-cytokératine (Pan-CK), la cadhérine E,
et/ou
les marqueurs mésenchymateux, dans lequel les marqueurs mésenchymateux sont choisis dans le groupe comprenant la cadhérine N et la vimentine,
et/ou
les marqueurs tumoraux, dans lequel les marqueurs tumoraux sont choisis dans le groupe comprenant AFP, KRT18, KRT19, TGFb, HIF1a, IGFBP1, IGFBP5, IGF, HGFAC, APC, VEGFA, PDGFA, FGFA, HIF1A, KDR1 (VEGFR), FGF2, HBsAg, ASGPR1, aSMA, Hep-Par-1,
et/ou
les marqueurs de résistance notamment C-Met,
et/ou
les marqueurs de cellules souches notamment CD133
et/ou
les marqueurs hématopoïétiques, dans lequel le marqueur hématopoïétique est choisi dans le groupe comprenant CD45 ou CD15,
et
d) ainsi d'identification d'un ou de plusieurs sous-groupes de CTC dans la population de CTC de cet échantillon biologique, dans lequel la composition du ou des sous-groupes de CTC est caractéristique d'une tumeur spécifique, dans lequel la tumeur est choisie dans le groupe constitué par le cancer du poumon non à petites cellules (CPNPC), le carcinome à cellules rénales métastatique (mRCC).

2. Procédé selon la revendication 1 comprenant l'étape d'isolement ou d'enrichissement en PBMC et en CTC provenant de l'échantillon biologique avant appauvrissement en cellules hématopoïétiques.

3. Procédé selon la revendication 1 ou 2 comprenant l'étape de test de la suspension cellulaire obtenue pour au moins un marqueur supplémentaire choisi parmi les marqueurs mésenchymateux, les marqueurs de transition épithéliale vers mésenchymateuse (EMT), les marqueurs de résistance notamment C-Met, les marqueurs de cellules souches, les marqueurs hématopoïétiques.

4. Procédé selon l'une des revendications précédentes, dans lequel les PBMC et les CTC sont isolées ou enrichies par centrifugation à densité de gradient.

5. Procédé selon l'une des revendications précédentes, dans lequel les cellules hématopoïétiques sont appauvries en utilisant des anticorps anti-CD45 et/ou anti-CD15, par exemple des billes immunomagnétiques revêtues d'anticorps anti-CD45 et/ou anti-CD15.

6. Procédé selon l'une des revendications précédentes comprenant l'étape d'exposition de l'échantillon biologique, des PBMC et des CTC isolées ou enrichies ou de la suspension cellulaire obtenue après appauvrissement en cellules hématopoïétiques à une composition pharmaceutique ou à un composé pharmaceutique.

7. Procédé selon l'une des revendications précédentes dans lequel la présence du ou des marqueurs dans la population de CTC est détectée par immunofluorescence, PCR ou FISH.
